# EUROPEAN PATENT APPLICATION

(11) **EP 3 822 351 A1**
(43) Date of publication of application: **19.05.2021**
(21) Application number: 19811579.2
(22) Date of filing: 31.05.2019
(51) Int. Cl.: C12N 15/113, C12N 15/63, C12N 5/077

(54) **MODIFIED NUCLEIC ACID INHIBITING MICRO RNA, AND USE THEREOF**

(30) Priority: 31.05.2018 KR 20180063047
(71) Applicant: Korea University Research and Business Foundation, Seoul 02841 (KR)
(72) Inventor: CHI, Sung Wook, Seoul 06362 (KR); JANG, Eun-Sook, Seoul 06362 (KR); SEOK, Hee Young, Seoul 06337 (KR)
(74) Representative: Dehns
(86) International application number: PCT/KR2019/006626
(87) International publication number: WO 2019/231299

(57) **Abstract**

The present invention relates to a modified nucleic acid that inhibits microRNA, and a use thereof. The modified nucleic acid that inhibits microRNA, according to the present invention, can strongly bond with microRNA by consecutively arranging at least two micro RNA target sites comprising a complementarily-bonding nucleotide sequence of a minimum of six on a micro RNA seed region, and, as a result of the insertion of an abasic spacer between the micro RNA target sites, not only effectively inhibits the micro RNA, but also has the benefit of the sequence inserted in the modified nucleic acid preventing inadvertent bonding with another micro RNA, and not only canonical target but also non-canonical target recognition of micro RNA such as a nucleation bulge site and G:A or G:U wobble arrangement sites can be used, and it is possible to selectively inhibit biological functions taking place due to non-canonical target recognition of micro RNA.

## Description

### [Technical Field]

The present invention relates to a modified nucleic acid inhibiting microRNA and a use thereof, and more particularly, to a modified nucleic acid inhibiting microRNA that complementarily binds to microRNA and a use thereof.

This application claims priority to and the benefit of Korean Patent Application No. 10-2018-0063047, filed on May 31, 2018, and Korean Patent Application No. 10-2019-0064767, filed on May 31, 2019, and the disclosure of the specification and drawings are incorporated herein by reference in their entirety.

### [Background Art]

RNA interference is a phenomenon of inhibiting gene expression at the post-transcriptional level, and is widely used as a technique for suppressing a desired target gene by artificially inducing gene silencing by introducing an RNA substance causing such a phenomenon into cells. The RNA interference is actually mediated by small RNA consisting of approximately 21 nucleotides called microRNA (miRNA), and miRNA exhibits a biological function by recognizing hundreds of target genes in mRNA through base pairing with a seed region (positions 1-8), particularly located at the 5' end of the miRNA, and inhibiting their expression.

Specifically, microRNA, which is a naturally-occurring RNA interference-induced nucleic acid, forms a complex with Argonaute protein, and then binds to a target through complementary base pairing. Here, in the case of miRNA found in animals, when a sequence of at least 6 consecutive bases in positions 1 to 8, most significantly, in positions 2 to 7 from the 5' end of the miRNA, called a seed region, is paired with target mRNA, the expression of the target mRNA is inhibited. Since the above-described type of base sequence most frequently occurs, it is defined as a canonical target of miRNA, and these types of complementary sequences are very important for the canonical biological function of miRNA. However, recently, due to a technique such as Ago HITS-CLIP that can experimentally sequence a miRNA target, it has been found that there are many non-canonical targets that go against the rule, rather than the recognition of a canonical target such as the seed region.

Since miRNA exhibits a biological function by recognizing target mRNA and inhibiting the expression thereof, it may be applied as a mechanism in which a nucleotide sequence that can complementarily pair with the seed region of miRNA while competing with the target mRNA, and thus the biological function induced by miRNA is inhibited (Ebert MS et al, Nat Methods. 2007 Sep;4(9): 721-6). Particularly, in the case of miRNA that has a disease-causing function, it can be applied as a therapeutic by specifically inhibiting the function. An inhibitor for the miRNA may generally include an almost completely complementary sequence to the entire sequence of miRNA, and thus is widely used in the mode of inhibiting the function by specific base pairing to miRNA. However, this type can inhibit specific miRNA, but not distinguish between a canonical target recognized by miRNA and a non-canonical target for inhibition. Therefore, when it is used as a drug, it may have an adverse effect that also inhibits all biologically required miRNA functions.

### [Disclosure]

### [Technical Problem]

The inventors had conducted earnest research to discover that an actual target-recognizing region in miRNA is a seed region between positions 1 to 8 from the 5' end, and significantly, between positions 2 to 7 from the 5' end, and thus it is possible to sufficiently bind with miRNA with only a complementary sequence to the seed region. It was confirmed that if there is a short 8-nt sequence, which is complementary to the seed region, the canonical target recognition of miRNA may be competitively inhibited, and when there are such short miRNA-binding sites repeatedly in the same molecule, they are able to more strongly pair with miRNA than the case that the entire sequence is complementary to miRNA. In fact, according to an Ago HITS-CLIP assay, it was observed that several short seed target sites are consecutively present at 3' UTR in target mRNAs of various miRNAs.

Therefore, based on the above descriptions, the present invention is directed to providing a modified nucleic acid that inhibits miRNA including a short sequence of two or more bases, which is able to be recognized as a miRNA target binding with miRNA according to a miRNA target recognition rule.

### [Technical Solution]

To attain the above-described object, the present invention provides a modified nucleic acid, which inhibits miRNA, having the following characteristics.

The present invention provides a modified nucleic acid inhibiting miRNA, in which
the modified nucleic acid consecutively includes at least two miRNA target site sequences, each consisting of at least 6 nucleotides,
the miRNA target site sequence may be one selected from the group consisting of
1) a sequence complementary to bases in positions 2 to 7 or 3 to 8 from the 5' end of miRNA;
2) a sequence complementary to bases in positions 2 to 7 from the 5' end of miRNA and having a bulge between the bases in positions 5 and 6; and
3) a sequence complementary to bases in positions 2 to 7 or 3 to 8 from the 5' end of miRNA, and having complementary G:A or G:U wobble base pairs, and
at least one base is substituted with an abasic spacer between the miRNA target site sequences.

In addition, the present invention provides a method of preparing a modified nucleic acid inhibiting miRNA, which includes: consecutively arranging one selected from the group consisting of 1) a sequence complementary to bases in positions 2 to 7 or 3 to 8 from the 5' end of miRNA,
2) a sequence complementary to bases in positions 2 to 7 from the 5' end of miRNA and having a bulge between the bases in positions 5 and 6, and
3) a sequence complementary to bases in positions 2 to 7 or 3 to 8 from the 5' end of miRNA, and having complementary G:A or G:U wobble base pairs; and
   substituting at least one base with an abasic spacer between the consecutively arranged base sequence.

In addition, the present invention provides a method of inhibiting miRNA, which includes introducing a modified nucleic acid inhibiting miRNA into cells *in vitro.*

In addition, the present invention provides a gene transfer system including a modified nucleic acid inhibiting miRNA.

In one embodiment of the present invention, the miRNA may be miRNA-1.

In another embodiment of the present invention, the modified nucleic acid inhibiting the miRNA may include a 5'-ACAUUCCA-3' (anti-miR-1-seed site; SEQ ID NO: 1) or 5'-AAAUUCCA-3' (anti-miR-1-7G:A seed site; SEQ ID NO: 2) sequence.

In still another embodiment of the present invention, the modified nucleic acid inhibiting miRNA may consecutively include at least two 5'-ACAUUCCA-3' sequences, and at least one abasic space is included before and after the sequence.

In yet another embodiment of the present invention, the modified nucleic acid inhibiting miRNA may consecutively include at least two 5'-AAAUUCCA-3' sequences, and at least one abasic space is included before and after the sequence.

In yet another embodiment of the present invention, the miRNA target site sequence may include 6 to 8 nucleotides complementary to the seed region of miRNA.

In yet another embodiment of the present invention, the seed region may be a sequence of bases in positions 1 to 8 from the 5' end of miRNA.

In yet another embodiment of the present invention, the abasic spacer may be one or more selected from the group consisting of an abasic ribonucleotide spacer (rSpacer, rSp) an abasic deoxyribonucleotide spacer (dSpacer), a C3 spacer and a C6 spacer, which contain no bases, and one of all spacer modifiers containing no bases.

### [Advantageous Effects]

A modified nucleic acid inhibiting miRNA according to the present invention can strongly bind with miRNA by consecutively arranging at least two miRNA target sites consisting of at least 6-nt sequence complementarily binding to the seed region of miRNA, and by introducing an abasic spacer between the miRNA target sites, has effects of not only effectively inhibiting miRNA, but also preventing competitive inhibition due to unintentional interactions between other miRNAs or RNA-binding proteins and a nucleotide sequence formed at the boundary region of the sequence introduced into the modified nucleic acid.

In addition, the modified nucleic acid can also use non-canonical target sites of miRNA, such as a nucleation bulge site and G:A or G:U wobble pair site of miRNA, as well as the canonical target sites, and therefore it is expected that only a biological function exhibited by non-canonical target recognition of miRNA can be selectively inhibited.

### [Description of Drawings]

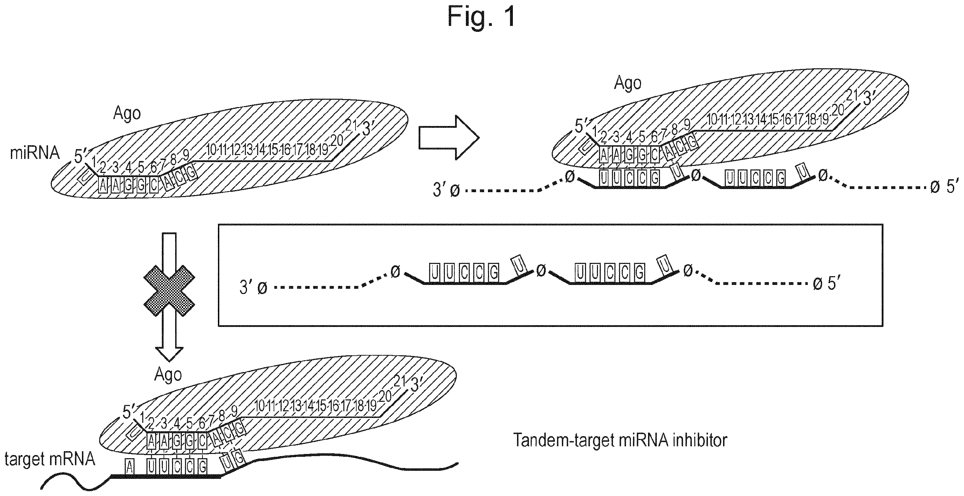

FIG. 1 is a diagram illustrating the action mechanism of a tandem-target miRNA inhibitor, which is a modified nucleic acid inhibiting miRNA according to the present invention.

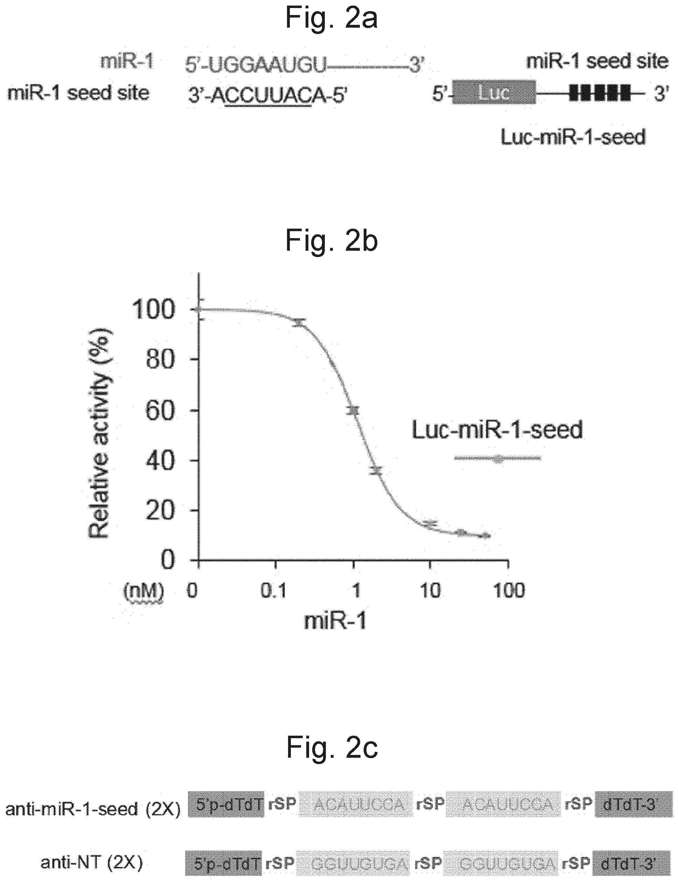

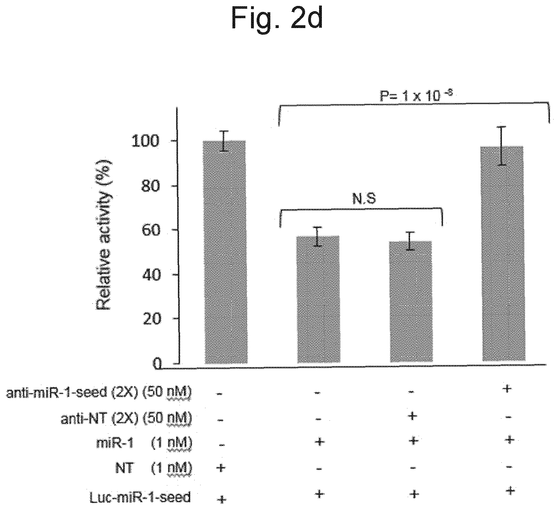

FIG. 2A shows miR-1 and a canonical seed target site of miR-1 (miR-1 seed site), and shows a luciferase reporter including the same (Luc-miR-1-seed), FIG. 2B shows the result of measuring the canonical seed target efficiency of miR-1 using a luciferase reporter, FIG. 2C shows a tandem-target miRNA inhibitor (anti-miR-1-seed(2x)) having two canonical seed target sites of miR-1 and a control (anti-NT(2x)), and FIG. 2D shows the result of confirming an inhibitory effect of the activity of miR-1 when the anti-miR-1-seed(2x) and the anti-NT(2x) of FIG. 2C are applied to miR-1 according to the present invention.

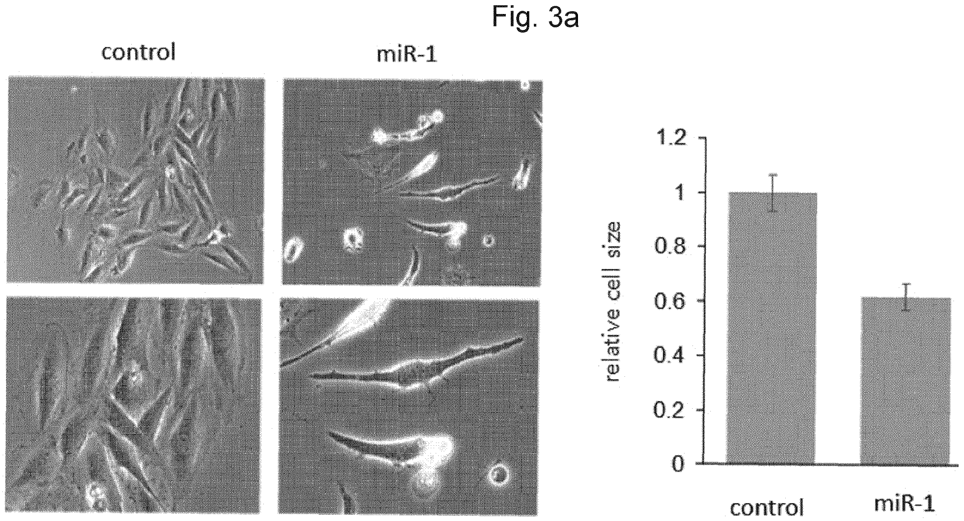

FIGS. 3A to 3C show the result of observing the effect of a tandem-target miRNA inhibitor inhibiting a canonical seed target site of miR-1 in a cardiomyoblast cell line, H9c2 cells, according to the present invention.

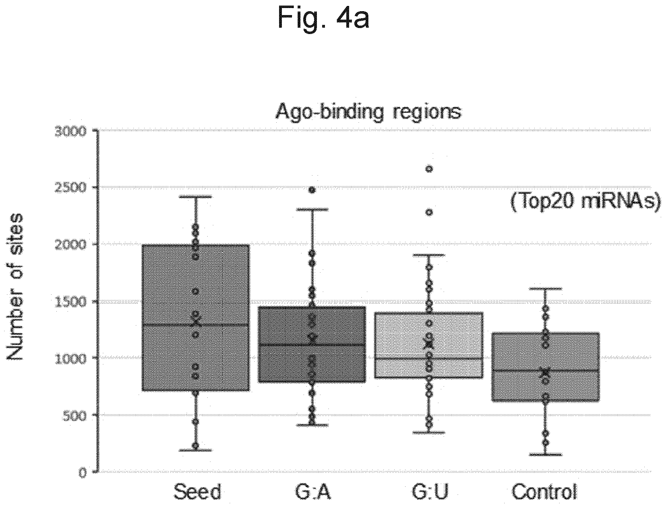

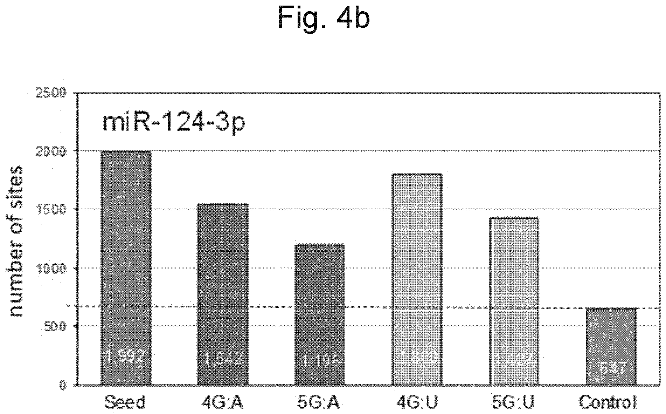

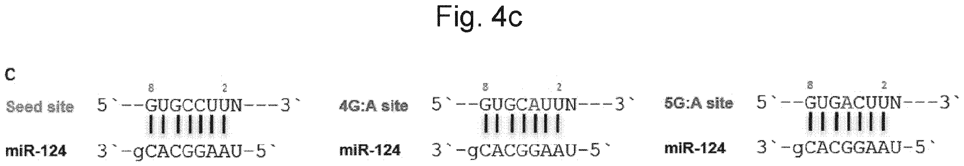

FIGS. 4A and 4B show the results obtained by data analysis for Ago HITS-CLIP, which is an miRNA target binding sequence, showing that miRNA can bind to target mRNA by atypically forming wobble base pairing (G:A wobble) through a seed region, and FIG. 4C shows a canonical seed site, and non-canonical G:A wobble seed sites through the G in position 4 (4G:A site) and G in position 5 (5G:A site) from the 5' end for miR-124.

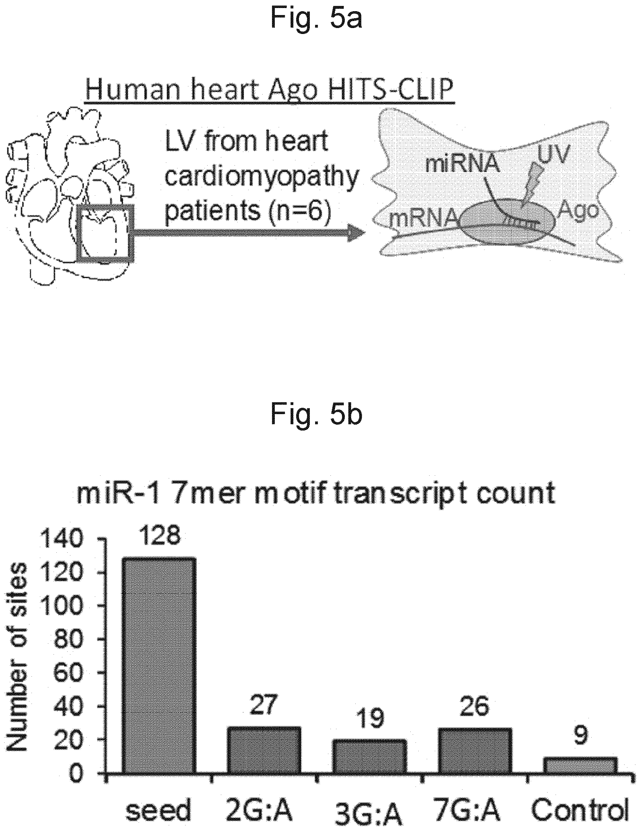

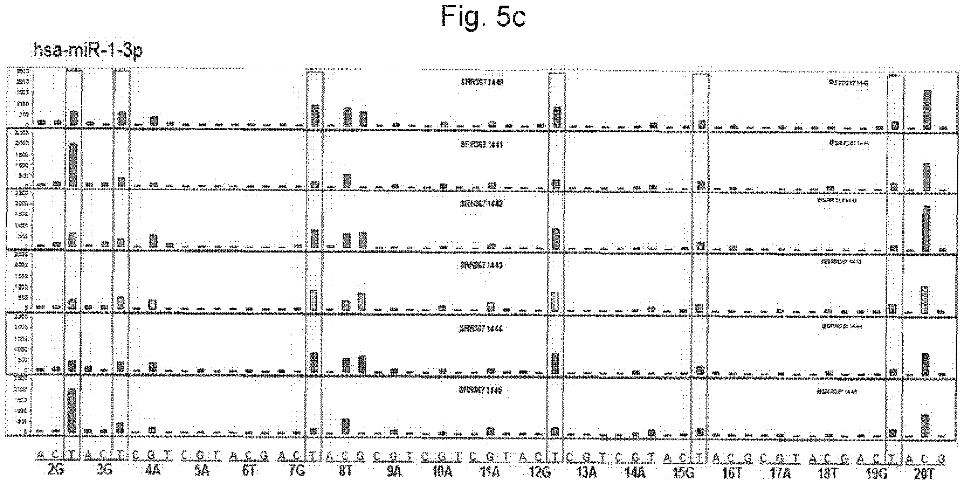

FIG. 5A schematically illustrates Ago HITS-CLIP data analysis performed on cardiac tissue of a cardiomyopathy patient, FIG. 5B shows that miR-1 binds to target mRNA by atypically forming wobble base pairing (G:A wobble pairs) through a seed region, and FIG. 5C shows that sequence variation of miR-1 occurs at the corresponding G:A site.

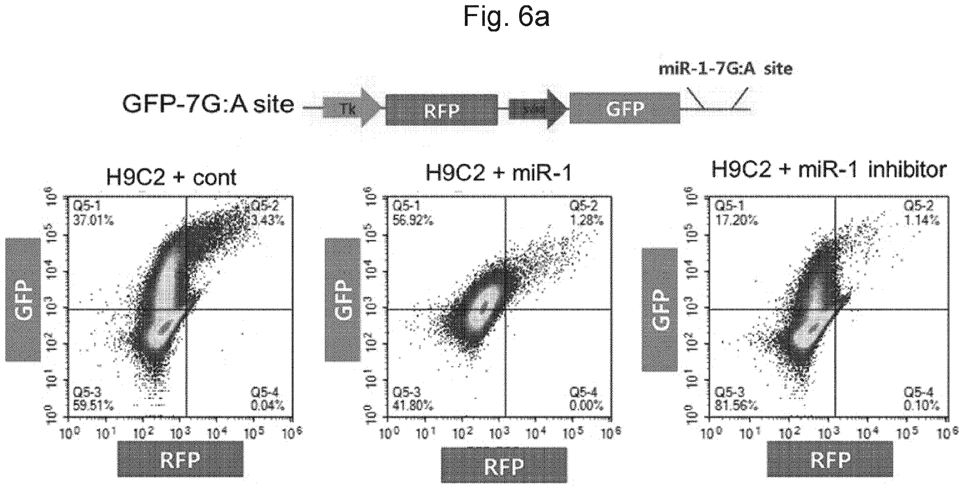

FIGS. 6A to 6D show the result of corresponding fluorescent protein reporter analysis, which confirms that miR-1 can suppress a target gene through the atypical G:A wobble seed target site of miR-1 found in FIG. 5 in a cardiomyoblast cell line, that is, H9c2 cells.

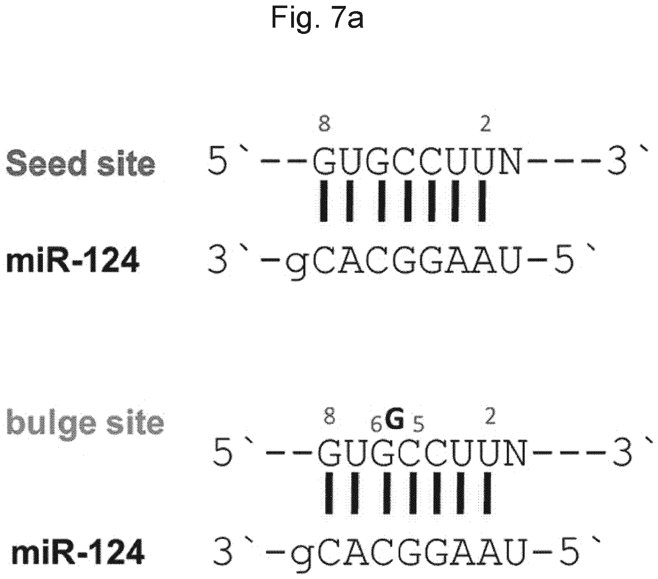

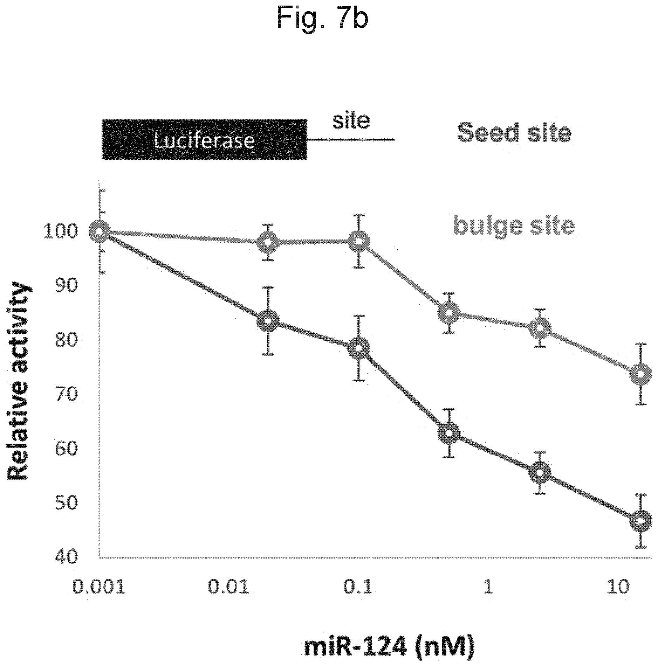

FIGS. 7A and 7B show the result of a luciferase reporter experiment on a non-canonical target recognition mode of miRNA, that is, bulge base pairing, for confirming that the target gene expression of miRNA is inhibited through non-canonical bulge base pairing of miRNA in miR-124, and suggests the fact that it is possible to develop an inhibitor thereby.

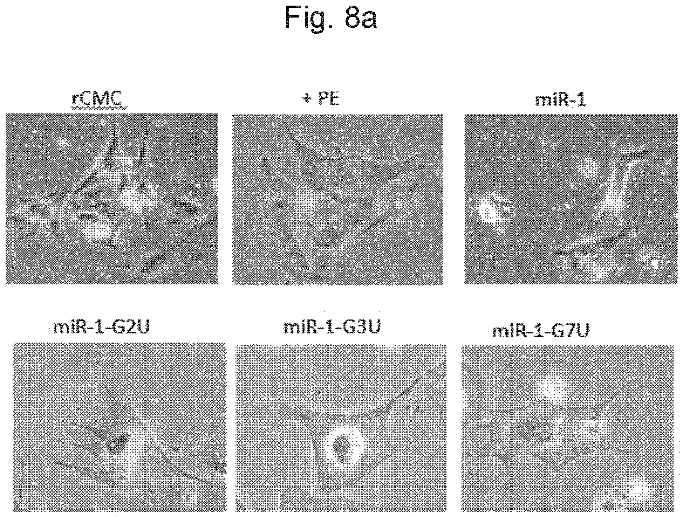

FIGS. 8A to 8C show the result of observing that miR-1 can induce cardiac hypertrophy, different from the function in miR-1, through the inhibition of a G:A wobble pair target in a seed sequence in primary rat cardiomyocytes.

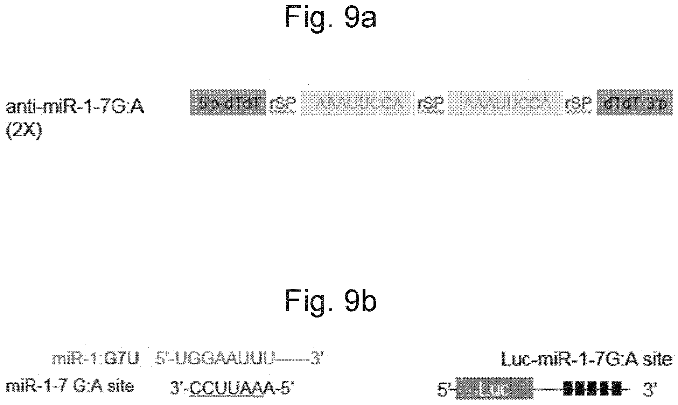

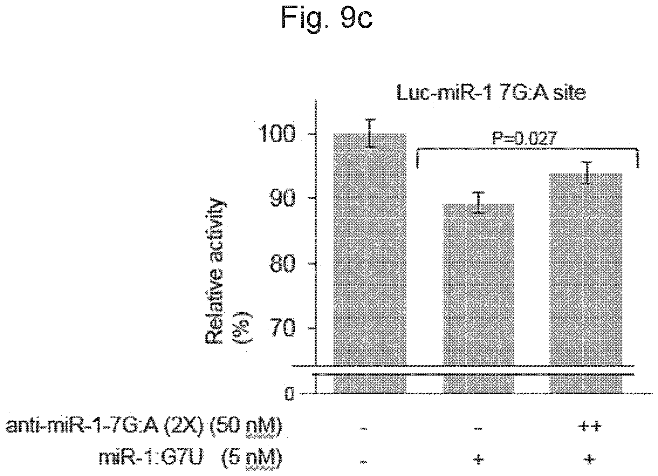

FIG. 9A shows a tandem-target miRNA inhibitor (anti-miR-1-7G:A(2x)) for inhibiting only non-canonical G:A wobble pair target recognition of miR-1, FIG. 9B shows a non-canonical G:A wobble seed target site of miR-1 (miR-1-7 G:A site) and a luciferase reporter gene including the same, and FIG. 9C shows the result of confirming whether the anti-miR-1-7G:A(2x) exhibits an inhibitory effect on miR-1:G7U suppressing a G:A wobble pair target gene.

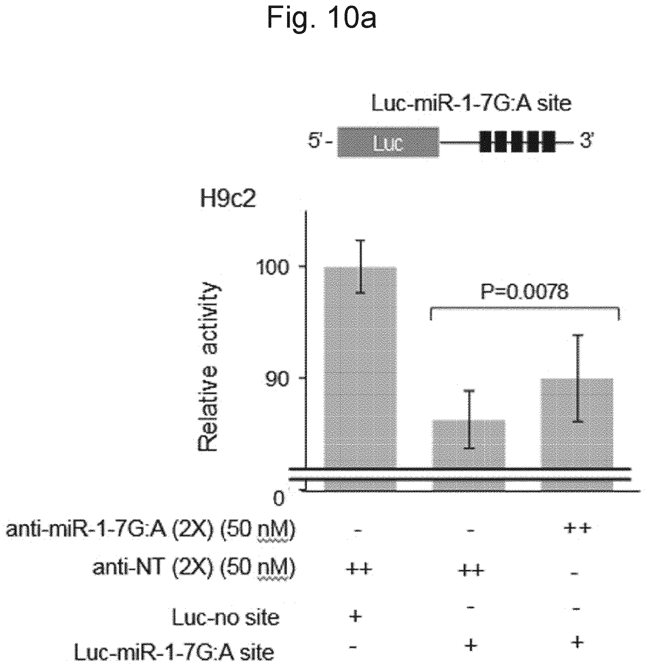

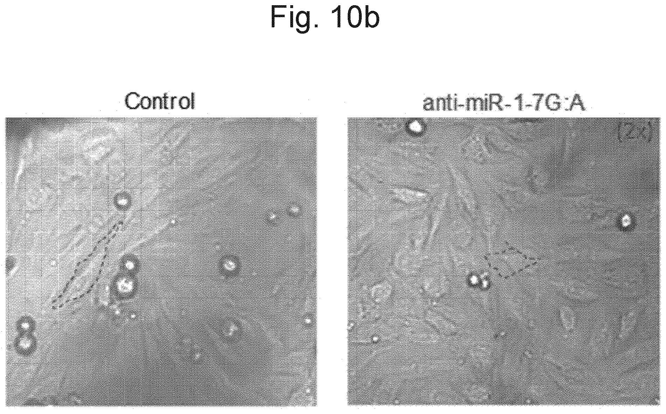

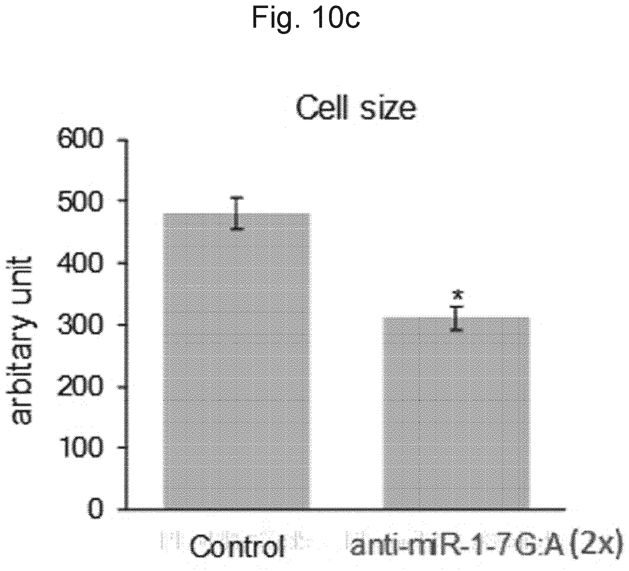

FIG. 10A shows the result of a luciferase reporter experiment, confirming that the tandem-target miRNA inhibitor (anti-miR-1-7G:A(2x)) of FIG. 9 can inhibit non-canonical G:A wobble pairing of miR-1 in a cardiomyoblast cell line (H9c2 cells), and FIGS. 10B and 10C show the phenomenon in which the size of cardiomyoblast cells (H9c2 cells) is reduced by anti-miR-1-7G:A(2x) as a biological effect caused thereby.

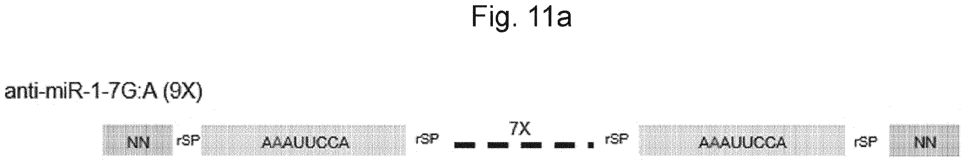

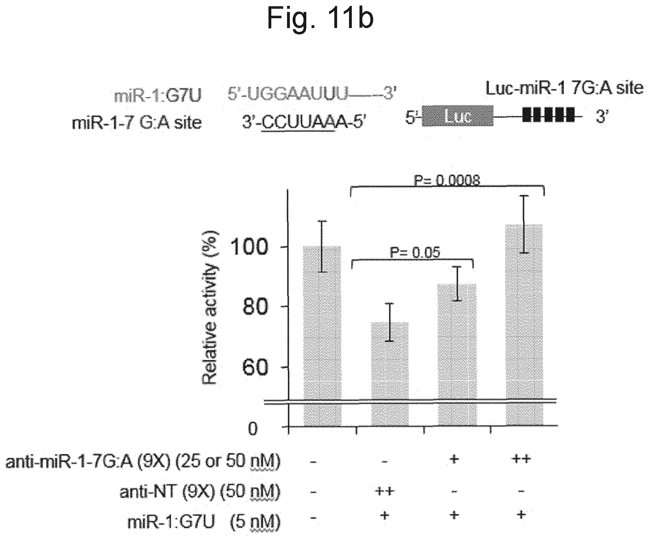

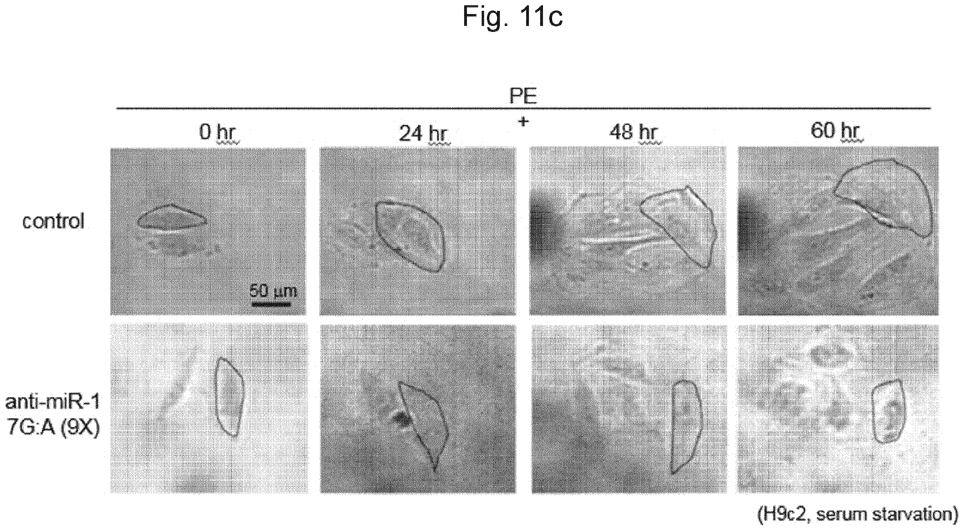

FIG. 11A shows a tandem-target miRNA inhibitor (anti-miR-1-7G:A(9x)) having 9 non-canonical G:A wobble sites of miR-1, FIG. 11B shows the result of a luciferase reporter experiment, showing that a gene suppression phenomenon caused by a non-canonical G:A wobble site can be prevented by applying anti-miR-1-7G:A(9x), and FIG. 11C shows that a cardiac hypertrophy-inducing phenomenon caused by a PE drug is inhibited in H9c2 cells.

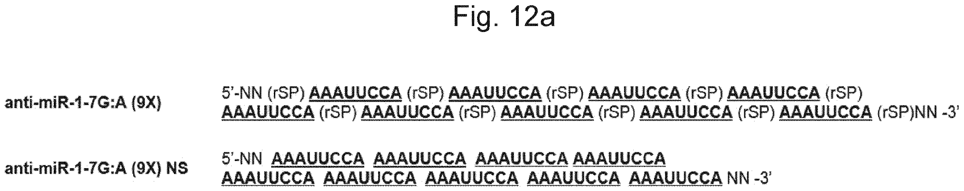

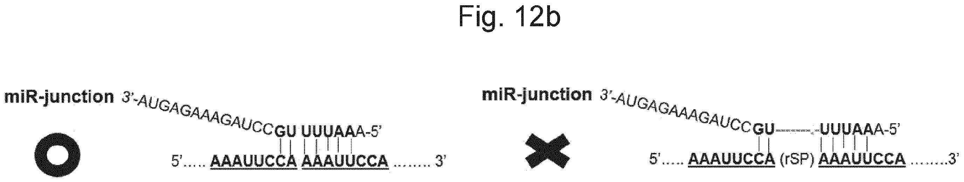

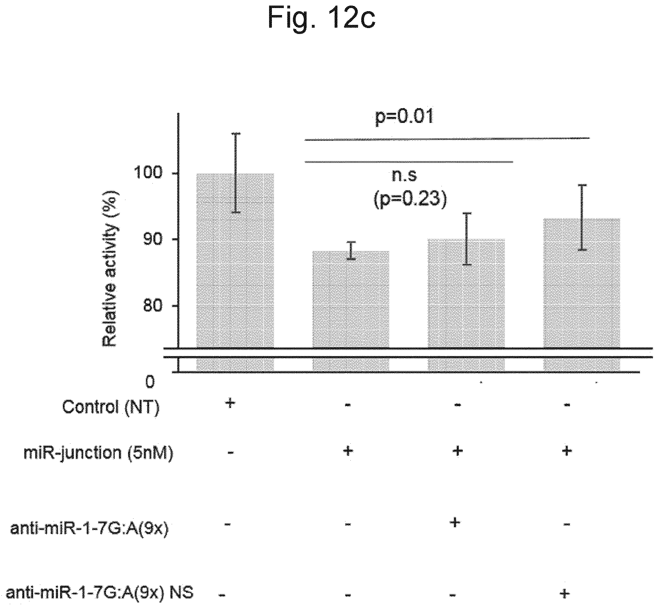

FIG. 12A shows sequences of a tandem-target miRNA inhibitor having an abasic spacer modifier between non-canonical G:A wobble sites of miR-1 (anti-miR-1-7G:A(9x)) and a control without an abasic spacer modifier (anti-miR-1-7G:A(9x) NS), FIG. 12B shows that, in the case of miRNA binding to a sequence of the paired region in a corresponding target site, an adverse effect in which the tandem-target miRNA inhibitor inadvertently inhibits the miRNA is shown, but when there is abasic spacer modifier in the target site, the adverse effect is not shown, and FIG. 12C shows the result of proving the fact shown in FIG. 12B by a luciferase reporter experiment.

### [Modes of the Invention]

The present invention may have various modifications and various embodiments, and thus specific embodiments are illustrated in the drawings and described in detail in the detailed description. However, it should be understood that the present invention is not limited to the specific embodiments, and includes all modifications, equivalents or alternatives within the idea and technical scope of the present invention. In the description of the present invention, detailed description of the related art will be omitted if it is determined that the gist of the present invention can be obscured.

Hereinafter, the present invention will be described in detail.

In the case of a conventional miRNA inhibitor, to increase an inhibitory effect, several sequences that can competitively bind to miRNA are arranged. Here, due to complementarity of base sequences newly produced at boundaries between the introduced multiple sequences, an adverse effect of inadvertently binding with different miRNAs may be exhibited. Therefore, as a result of the effort to continuously increase the multiplicity in pairing of the corresponding sequences and develop an miRNA inhibitor that can solve the adverse effect caused by the boundary sequence, the inventors invented a modified nucleic acid including at least two consecutive miRNA target sites having a complementary sequence to a seed region of miRNA and inhibiting miRNA by introducing an abasic spacer, where there is no base pairing due to no bases, between the miRNA target sites, and thus the present invention was completed.

The present invention provides a modified nucleic acid inhibiting miRNA, in which the modified nucleic acid consecutively includes at least two miRNA target site sequences, each consisting of at least 6 nucleotides,
the miRNA target site sequence may be one selected from the group consisting of
1) a sequence complementary to bases in positions 2 to 7 or 3 to 8 from the 5' end of miRNA,
2) a sequence complementary to bases in positions 2 to 7 from the 5' end of miRNA and having a bulge between the bases in positions 5 and 6, and
3) a sequence complementary to bases in positions 2 to 7 or 3 to 8 from the 5' end of miRNA, and having complementary G:A or G:U wobble base pairs, and
at least one base is substituted with an abasic spacer between the miRNA target site sequences.

In the present invention, the modified nucleic acid inhibiting miRNA may include a 5'-ACAUUCCA-3' or 5'-AAAUUCCA-3' sequence. However, as long as the miRNA target site sequence corresponding to any one of 1) to 3) is included, the sequence is not limited thereto.

In the present invention, the modified nucleic acid inhibiting miRNA may be a modified nucleic acid consecutively including at least two 5'-ACAUUCCA-3' sequences, and at least one abasic spacer before and after the sequence, or a modified nucleic acid consecutively including at least two 5'-AAAUUCCA-3' sequences and at least one abasic spacer before and after the sequence.

There is no limitation to the type of miRNA according to the present invention, and according to an exemplary embodiment of the present invention, the miRNA may be miRNA-1.

In the present invention, the miRNA target site sequence may include at least 6 nucleotides, preferably 21 or less nucleotides, and most preferably, 6 to 8 nucleotides, but the present invention is not limited thereto.

Since miRNA actually recognizes only at least 6 base pairs in positions 1 to 8 from the 5' end thereof, even only a sequence complementary to the base sequence in positions 1 to 8 can sufficiently bind with miRNA, and when the miRNA target site sequence includes 6 to 8 nucleotides, only the canonical target recognition of miRNA may be competitively inhibited.

In the present invention, the modified nucleic acid inhibiting miRNA may consecutively include at least two miRNA target site sequences, and as the two or more miRNA target site sequences are consecutively included, it is better. For example, 100 or less miRNA target site sequences may be included, and preferably, 2 to 70, 2 to 50, 2 to 30, 2 to 9, 9 to 30, 9 to 50, or 9 to 70 miRNA target site sequences are consecutively included, but the present invention is not limited thereto. Compared with when only one miRNA target site sequence is included, when two miRNA target site sequences are consecutively included, they may more strongly bind with miRNA. In one embodiment of the present invention, compared with when the modified nucleic acid inhibiting miRNA consecutively includes two miRNA target site sequences, when consecutively including 9 miRNA target site sequences, it was confirmed that a more improved inhibitory effect is exhibited (refer to Example 4-2).

The "consecutively (included)" means that miRNA target site sequences are linearly included in the single strand of a modified nucleic acid, and may also be included as spaced-apart from each other.

In the present invention, at least one base, preferably more than one base, and for example, 10 or less bases may be substituted with an abasic spacer and connected between the consecutively arranged miRNA target site sequences, but the present invention is not limited thereto.

Since miRNA may sufficiently bind with only matched 6 to 8 bases in positions 1 to 8 from the 5' end, a sequence introduced into the modified nucleic acid inhibiting miRNA may work by accidental base-pairing with an unintentional different miRNA. When one base in the miRNA target site sequence is substituted with the abasic spacer, the miRNA target site sequences may be prevented from combining to form a new site, thereby preventing the sequence introduced into the miRNA target site from inadvertently binding with different miRNAs.

The term "abasic spacer" used herein may be a substitute for maintaining a space instead of a single nucleotide, and may connect neighboring nucleotides in the manner that can maintain the space. The abasic space is preferably an organic compound, more preferably, a phosphate group (-H₂PO₄) or a sulfuric acid group (-H₂PSO₄)-containing (linked) hydrocarbon chain, and most preferably, an alkyl group having at least three carbon atoms (C3 spacer) or an alkyl group having at least 6 carbon atoms (C6 spacer), but the present invention is not limited thereto. In addition, the abasic spacer may include an abasic nucleotide, and such an abasic nucleotide-type spacer generally refers to a single nucleotide analogue having no base, and refers to all types of modification that cannot bind with all other biological bases comprehensively originating from RNA, including an abasic deoxyribonucleotide spacer (dSpacer) and an abasic ribonucleotide spacer (rSpacer). According to an exemplary embodiment of the present invention, the abasic spacer may be an rSpacer molecule having an Ribo nucleotide as a backbone.

In the present invention, the miRNA target site sequence is complementary to the base sequence of the seed region of miRNA.

In the present invention, the seed region refers to a sequence between positions 1 to 8 from the 5' end of miRNA.

The modified nucleic acid inhibiting miRNA according to the present invention can be used as not only a canonical target of miRNA, but also a non-canonical target recognition-type nucleation bulge site and a G:A or G:U wobble base pair site.

Here, the non-canonical target recognition refers to recognition as a target of miRNA even if the seed region of miRNA is not exactly complementary when miRNA binds with target mRNA.

In the present invention, the nucleation bulge site refers to bulging bases complementary to the 5^{th} and 6^{th} bases from the 5' end of miRNA, and a gene having a complementary relationship with all bases until the 5^{th} base from the 5' end of miRNA and recognized by the 6^{th} base (pairing with the 6^{th} base from the 5' end of miRNA) refers to the case in which the immediately consecutive base is looped out in a bulge form, and the base having a complementary relationship with miRNA is the base recognized by the 6^{th} base from the 5' end of the miRNA, rather than a base immediately consecutive with the base having a complementary relationship to the 5^{th} base at the 5' end of the miRNA.

The G:A or G:U wobble base pairing means that bases of miRNA and the target gene recognized by miRNA have G:A or G:U base pairing, in addition to the complementary pairing of A:U, G:C, C:G or U:A.

For example, when miRNA and the target gene recognized by miRNA are in a G:A wobble pair relationship, the pair of a base of specific miRNA : a base of a target gene recognized by the specific miRNA is G:A, when the miRNA and the target gene recognized by the miRNA are in a G:U wobble relationship, the pair of a base of specific miRNA: a base of a target gene recognized by the specific miRNA is G:U.

In addition, when miRNA and the target gene recognized by miRNA are in a G:A wobble pair relationship, a base of a target gene recognized by the specific miRNA: a base of specific miRNA: a base of a modified nucleic acid inhibiting specific miRNA may be A:G:A, and when miRNA and the target gene recognized by miRNA are in a G:U wobble pair relationship, a base of a target gene recognized by the specific miRNA: a base of specific miRNA: a base of a modified nucleic acid inhibiting specific miRNA may be U:G:U.

In the present invention, a modified nucleic acid inhibiting miRNA may be a nucleic acid such as anti-sense RNA, LNA, PNA, UNA, siRNA, miRNA, shRNA, DsiRNA, 1siRNA, ss-siRNA, asiRNA, piRNA or endo-siRNA and a nucleic acid derivative, but the present invention is not limited thereto.

In addition, the present invention provides a method of preparing a modified nucleic acid inhibiting miRNA, which includes:
consecutively arranging one selected from the group consisting of 1) a sequence complementary to bases in positions 2 to 7 or 3 to 8 from the 5' end of miRNA,
   2) a sequence complementary to bases in positions 2 to 7 from the 5' end of miRNA and having a bulge between the bases in positions 5 and 6, and
   3) a sequence complementary to bases in positions 2 to 7 or 3 to 8 from the 5' end of miRNA, and having complementary G:A or G:U wobble base pairs; and
substituting at least one base with an abasic spacer between the consecutively arranged base sequences.

Regarding the method of preparing a modified nucleic acid inhibiting miRNA, the contents described above overlap, and therefore, detailed descriptions thereof will be omitted.

In addition, the present invention provides a method of inhibiting miRNA, which includes introducing a modified nucleic acid inhibiting miRNA into cells *in vitro.*

In the present invention, the type of "cells" include vertebrates, for example, mammals including a human (a human, a monkey, a mouse, a rat, a hamster, a cow, etc.), birds (a chicken, an ostrich, etc.), amphibians (a frog, etc.), and fish, or invertebrates, for example, insects (a silkworm, a moth, a fruit fly, etc.), plants, or microorganisms such as yeast, and preferably, mammals including a human, but the present invention is not limited thereto.

In the present invention, the "introduction" refers to modification of the genotype of cells by injecting a nucleic acid into the cells, and there is no particular limitation to the method of injecting a nucleic acid into cells. The introduction may be performed by methods well known to those of ordinary skill in the art to which the present invention belongs, such as transfection using calcium phosphate (Graham, FL et al., Virology, 52:456(1973)), transfection by DEAE dextran, transfection by microinjection (Capecchi, MR, Cell, 22:479(1980)), transfection by a cationic lipid (Wong, TK et al., Gene, 10:87(1980)), electroporation (Neumann E et al., EMBO J, 1:841(1982)), transduction or transfection as disclosed in the documents (Basic methods in molecular biology, Davis et al., 1986, and Molecular cloning: A laboratory manual, Davis et al., 1986), and preferably, transfection. Transfection efficiency may vary greatly depending on a cell type as well as cell culture conditions and a transfection reagent.

In addition, the present invention provides a gene transfer system including the modified nucleic acid inhibiting miRNA.

The "gene transfer" refers to the delivery of a gene into cells, and may have the same meaning as intracellular transduction of a gene. At a tissue level, the term "gene transfer" may have the same meaning as the spread of a gene. In addition, the gene transfer system of the present invention may be formed in various forms, such as a naked recombinant DNA molecule, a plasmid virus vector and a liposome or niosome containing the naked recombinant DNA molecule or plasmid, but the present invention is not limited thereto.

In addition, the present invention provides a composition for regulating cardiac hypertrophy, which includes a modified nucleic acid inhibiting miRNA. In an exemplary embodiment of the present invention, it was confirmed that, when cardiac hypertrophy was induced in cardiomyocytes, the size of cardiomyocytes was reduced by canonical target recognition of miR-1, and increased to a level similar to cardiac hypertrophy-induced cells by non-canonical target recognition through G:A wobble pairing (refer to Example 3).

The modified nucleic acid inhibiting miRNA according to the present invention may competitively inhibit binding of miRNA with target mRNA due to complementary binding to miRNA, and the action mechanism of a tandem-target miRNA inhibitor, which is the modified nucleic acid inhibiting the miRNA, is illustrated in FIG. 1.

In another embodiment of the present invention, it was confirmed that miRNA can inhibit a target gene through G:A wobble pairing in a seed sequence, and the non-canonical nucleation bulge site of miRNA was inhibited by a luciferase reporter (refer to Example 2).

In still another embodiment of the present invention, it was confirmed that miRNA can exhibit a different biological function through G:A wobble pair target inhibition in a seed sequence (refer to Example 3).

In yet another embodiment of the present invention, the effect of inhibiting a non-canonical G:A wobble pairing function of miRNA by a tandem-target miRNA inhibitor was confirmed (refer to Example 4).

In yet another embodiment of the present invention, the effect of eliminating an adverse effect caused by a linker sequence between target sites through the introduction of a spacer modifier into a tandem-target miRNA inhibitor was confirmed (refer to Example 5).

Hereinafter, preferable examples and experimental examples of the present invention will be described in detail with reference to the accompanying drawings. However, these examples and experimental examples are merely provided to exemplify the present invention, and it will not be construed that the scope of the present invention is not limited thereto.

### Example 1. Confirmation of effect of inhibiting canonical seed binding function of miRNA by tandem-target miRNA inhibitor

### 1-1. Method of preparing tandem-target miRNA inhibitor

As shown in FIG. 1, 5'-UAAGGCACG-3', which is the base sequence in positions 1 to 8 from the 5' end of the base sequence of Argonaute protein-bound miRNA, is a seed base sequence of the corresponding miRNA (example for miR-124), and the miRNA complementarily binds to target mRNA using this sequence. Here, when a tandem-target miRNA inhibitor, which is a modified nucleic acid inhibiting miRNA, is prepared by introducing two or more short 8-nt target sequences that are able to complementarily pair with an miRNA seed, the miRNA seed (5'-UAAGGCACG-3') and a 5'-UGCCUU-3' sequence of the tandem-target miRNA inhibitor are completely complementary, thereby relatively inhibiting pairing with actual target mRNA bases and suppressing the canonical target inhibitory function of the miRNA. The tandem-target miRNA inhibitor consecutively contains two or more corresponding base sequences (5'-UGCCUU-3') for stronger and more efficient base pairing, making it possible to have one or more base pairs between the miRNA seed and the tandem-target miRNA inhibitor. In addition, by connecting the consecutively-arranged base sequences (5'-UGCCUU-3') with an abasic spacer, off-target repression caused by a newly generated consecutive sequence at the boundary between the corresponding base sequences is able to be fundamentally prevented.

### 1-2. Confirmation of effect of tandem-target miRNA inhibitor on canonical seed target site of miR-1

To confirm the effect of the tandem-target miRNA inhibitor shown in Example 1-1, the inhibitor was applied to miR-1. Here, to experimentally confirm the target inhibitory effect of miR-1, a luciferase reporter assay was conducted. To this end, as shown in FIG. 2A, as a canonical seed target site of miR-1 (miR-1 seed site), five target site sequences each including a sequence consecutively complementary to nucleotides in positions 2 to 8 from the 5' end of miR-1, which is the minimum standard as a canonical seed target site of miR-1 (miR-1 seed site), were consecutively arranged at the 3' end of a luciferase reporter gene, thereby forming a luciferase reporter vector (psi-check2, Promega; Luc-miR-1-seed).

Subsequently, to measure the functionality of the luciferase reporter vector formed as above, along with the suppressive ability of a gene having a canonical target seed site according to a change in miR-1 concentration using the luciferase reporter vector, miR-1 were introduced into cells at various concentrations (0, 0.01, 0.1, 0.5, 2.5, 15 nM), and an inhibitory concentration 50 (IC₅₀) was measured as the activity of a luciferase. Its effect was investigated by co-transfection of the corresponding concentration of miR-1 and the luciferase reporter vector formed by the above-described method into HeLa cells (ATCC CCL-2) using the Lipofectamine 3000 reagent (Invitrogen) according to the manufacturer's protocol. Here, the miR-1 was chemically synthesized with two nucleotide sequences, such as a guide strand and a passenger strand, by Bioneer, separated by HPLC, and then duplexed by the method provided by the manufacturer. HeLa cells were incubated in Dulbecco's modified Eagle's medium (Invitrogen) supplemented with 10% fetal bovine serum (FBS), 100 U/ml penicillin and 100 µg/ml streptomycin, and during transfection, the cells were incubated in an antibiotic-free complete medium. Twenty-four hours after transfection, the luciferase activity was measured using a dual-luciferase reporter assay system (Promega) according to the manufacturer's protocol, and here, the measurement of *Renilla* luciferase activity was repeated at least three times using a Glomax Luminometer (Promega), and calculated by normalization by firefly luciferase activity.

As a result, as shown in FIG. 2B, it was able to be seen that the IC₅₀ of miR-1 is 2.2 nM, and the target gene suppression by miR-1 can be precisely measured using the luciferase vector formed as above.

To confirm the effect of the tandem-target miRNA inhibitor described in Example 1-1, a tandem-target miRNA inhibitor (anti-miR-1-seed(2x)) for inhibiting a seed target site, which is a canonical miR-1 target, was chemically synthesized by Bioneer to have a sequence shown in FIG. 2C, and then separated by HPLC. Here, the base sequence and composition of the inhibitor are as follows (anti-miR-1-seed(2x): 5' p-dTdT (rSP) ACAUUCCA (rSP) ACAUUCCA (rSP)dTdT-3', SEQ ID NO: 3). Specifically, since, in the base sequence of miR-1 (5' p-UGGAAUGUAAAGAAGUAUGUAU-3', SEQ ID NO: 4), the base sequence of the seed region recognizing a canonical target is 5'-UGGAAUGU-3', the tandem-target miRNA inhibitor has two repeated base sequences (5'-ACAUUCCA-3') having completely complementary pairing with the base sequence of the miR-1 seed region. By connecting these canonical target sequences with rSpacer (rSP), which is an abasic ribonucleotide with no base, an off-target effect caused by the consecutive creation of canonical target sites at the boundary between two adjacent target sequences was prevented.

The sequence of the tandem-target miRNA inhibitor (anti-miR-1-seed) for the miR-1 seed target formed by the above-described method is shown in FIG. 2C. In addition, to prevent degradation of the 5' end and 3' end of the tandem-target miRNA inhibitor in cells due to the attack of an exonuclease, two nucleotides at either end were synthesized to have deoxynucleotides, such as thymine (dT). At the same time, as a control, a tandem-target miRNA inhibitor (anti-NT (2x)) was designed with the following sequence (anti-NT (2x): 5' p-dTdT (rSP) GGUUGUGA (rSP) GGUUGUGA (rSP) dTdT-3', SEQ ID NO: 6) using a canonical seed target site of cel-miR-67 (NT; non-targeting: 5'-UCACAACCUCCUAGAAAGAGUAGA -3', SEQ ID NO: 5), which is C. *elegans*-specific miRNA, in the same manner as described above. The tandem-target miRNA inhibitor was also chemically synthesized by Bioneer, and separated by HPLC.

In addition, through the IC₅₀ assay shown in FIG. 2B, 1 nM miR-1 exhibiting 40% inhibition of target gene expression and 50 nM of the inhibitor (anti-miR-1-seed(2x) or anti-NT (2x)) were co-transfected into HeLa cells (ATCC CCL-2) using the Lipofectamine 3000 reagent (Invitrogen) according to the manufacturer's protocol so as to investigate the effect of anti-miR-1-seed(2x) on inhibiting the canonical seed target suppression of miR-1.

As a result, as shown in FIG. 2D, the luciferase activity observed by transfection of 1 nM miR-1 was approximately 50% (similar to that expected from IC₅₀), that is, a relative luciferase activity (%) of 56.58% +/- 4.27, calculated based on the value with non-transfection of miR-1. The relative luciferase activity (%) of an experimental group into which the same concentration of miR-1 was introduced with 50 nM anti-NT (2x) was 54.07% +/- 3.89, which was not significantly different from the value of luciferase activity regulated by miR-1, and therefore, an anti-NT (2x)-mediated suppression effect was not able to be observed. On the other hand, the relative luciferase activity percentage of an experimental group into which miR-1 and anti-miR-1-seed(2x) were simultaneously introduced was 96.43 +/- 8.44, an effect of completely recovering the luciferase activity value regulated by miR-1 was confirmed, which was observed to be statistically significant (p=1.0 x 10⁻⁸). From the above, it was able to be confirmed that the anti-miR-1-seed(2x) can specifically prevent the suppression of the canonical seed target gene induced by miR-1 completely.

### 1-3. Confirmation of inhibitory effect of miR-1 on size reduction of cardiomyocytes by tandem-target miRNA inhibitor in cardiomyocytes

To verify whether the tandem-target miRNA inhibitor which was prepared by the method as in Example 1-1 and whose effect as a miRNA inhibitor was confirmed as in Example 1-2 can inhibit the biological function of miRNA, the inhibitor was applied to a canonical seed-binding site of miR-1. First, to detect miR-1 in cardiomyocytes, a cardiomyoblast cell line (H9c2) was purchased from the American Type Culture Collection (ATCC), miR-1 was introduced thereinto, and then it was observed whether cell size reduction, which is a known function of miR-1 in cardiomyocytes, had been induced. Here, the H9c2 cells were incubated in a Dulbecco's modified Eagle's medium (Invitrogen) supplemented with 10% FBS, 100 U/ml penicillin and 100 µg/ml streptomycin, the miR-1 was prepared by chemically synthesizing double strands by Bioneer according to the human miR-1 sequence (MIMAT0000416) shown in the miRNA database, miRBase (www.mirbase.org), and separating it by HPLC. In addition, a control was synthesized to be the same as the sequence of cel-miR-67 (MIMAT0000039), which is miRNA of C. elegans. Afterward, the synthesized double-stranded miRNA was prepared in a duplex of a guide strand and a passenger strand according to the method provided by the manufacturer, and the miRNA introduction into H9c2 cells was performed by transfecting 50 mM of miRNA using an RNAiMAX reagent (Invitrogen) according to the manufacturer's protocol, and its effect was investigated 48 hours after transfection.

As a result, as shown in the images on the left side of FIG. 3A, compared with the control, it was observed that the sizes of the miR-1-introduced H9c2 cells are reduced. In addition, as a result of quantitative analysis of 100 or more of the cells using the ImageJ program (NIH), as shown in the graph on the right side of FIG. 3A, it was observed that the sizes of the miR-1 duplex-introduced cells are reduced to approximately 60% of the control cells.

In addition, after the function of reducing the size of cardiomyocytes by miR-1 was confirmed in H9c2 cells as above, as in Example 1-2, two base sequences that can complementarily pair to the seed region of miR-1 were formed with a tandem-target miRNA inhibitor (anti-miR-1-seed(2x)) (FIG. 2C), and then transfected into a cardiomyoblast cell line (H9c2) to carry out an experiment of confirming whether the miR-1 function was inhibited.

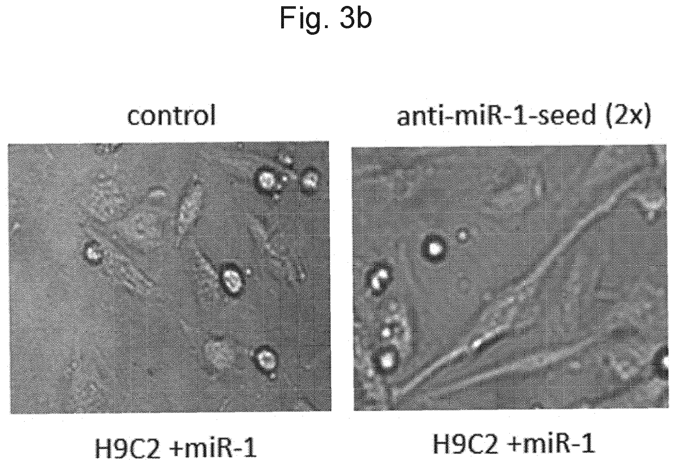

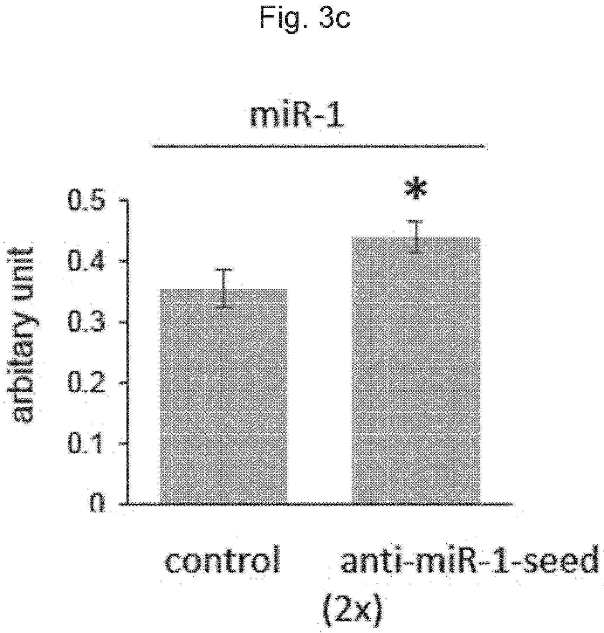

While the miR-1-induced reduction in cell size is still observed in the control (NT) and the case of co-transfection with miR-1, when the anti-miR-1-seed(2x) and miR-1 were introduced into H9c2 cells by co-transfection at a concentration of 25 nM, respectively, in the same manner as described above, as shown in FIGS. 3B and 3C, it was confirmed that such an effect is inhibited in the case of the anti-miR-1-seed(2x).

As described above, a phenomenon in which the anti-miR-1-seed(2x) inhibits the miR-1 function of reducing the size of cardiomyocytes, indicating that a canonical target inhibitory function of miRNA can be effectively inhibited through consecutive pairing of two or more canonical binding sites, each consisting of 8 bases recognized as a seed region.

### Example 2. Discovery and verification of non-canonical target site allowing wobble base pairing in seed region of miRNA

### 2-1. Detection of non-canonical G:A wobble pair site of miRNA expressed in cerebral cortex through Ago HITS-CLIP assay

As a method of analyzing an miRNA target at a transcriptome level, an experimental method called Ago HITS-CLIP had been developed and widely used, and the Ago HITS CLIP assay is a method of inducing a covalent bond between RNA and the Argonaute protein in cells by UV irradiation of cells or a tissue sample, separating the RNA-Argonaute complex formed thereby through immunoprecipitation using an antibody specifically recognizing Argonaute, and analyzing the separated RNA through next-generation sequencing. The base sequence data obtained thereby may not only identify target mRNA of miRNA through bioinformatics analysis, but also precisely analyze its binding site and sequence. Ago HITS-CLIP was first applied to mouse cerebral cortex tissue to perform mapping for the miRNA target in the brain tissue. Since then, this method has been applied to various tissues. Through numerous Ago HITS-CLIP assays, miRNA binding sites in various tissues were identified, and it was found that there are many non-canonical binding sites which make similar miRNA seed binding particularly in the above-mentioned target sequence, but different binding in the sequence of target mRNA.

It was observed that some of the known non-canonical binding rules are present as wobbles through G:U base pairing, other than the conventional known base pairings. The wobble base pairing is found in specific RNA, different from the conventional base pairing, and it was shown that the well-known G:U wobble pair may also be formed in a non-canonical target of miRNA. However, compared with this, a G:A wobble pair which has weak binding and is not often found may form base pairing in specific RNA, but other than this, has not been investigated at all. However, in the case of the seed sequence of miRNA, since the free energy of base pairing is structurally stabilized by the Argonaute protein, generally weak G:A base pairing may be relatively significant.

Therefore, first, to confirm whether wobble base pairing including a G:A pair is present in the miRNA target of the human brain tissue, Ago HITS-CLIP data from the gray matter of the brain tissue after death was analyzed. This analysis was performed by pairing an RNA sequence that had been bound with Argonaute-miRNA according to the method used in Ago HITS-CLIP development to a human genomic sequence by Bowtie2 using a FASTQ file. In addition, the corresponding sequencing result paired to a human miRNA sequence at the same time so as to also analyze 20 types of miRNAs (top 20 miRNAs) frequently binding to the Argonaute protein in the corresponding brain tissue was analyzed. Finally, for the top 20 miRNAs identified by the above-described method, a canonical target site having a complementary sequence to a corresponding seed sequence, which is the sequence of 2^{nd} to 8^{th} bases from the 5' end, was examined. Here, as shown in FIG. 4A, it was observed that a considerably large number of canonical seed target sites are found at miRNA binding sites (median: 1292.5 sites, error range: +/- 706.62). Afterward, from the Ago HITS-CLIP data, whether non-canonical target sites that are bound with G:U or G:A wobble pairing are present in such an miRNA seed base sequence was examined. Here, for analysis, a target sequence which cannot have complementary pairing of bases due to the identical base sequence to the miRNA seed base sequence, was used as a control.

As a result, as shown in FIG. 4A, all of G:A wobble pairs pair-allowed target sites (median: 1119.5 sites, error range: +/- 526.48) and G:U wobble pair-allowed target sites (median: 995 sites, error range: +/- 523.22) showed higher distribution than the control (median 891 sites, error range: +/- 410.71), indicating that, particularly, there are approximately 1.1 times more miRNA target sites having a G:A wobble pair than those having a G:U wobble pair.

In addition, miR-124 specifically expressed in brain tissue has two G bases in positions 4 and 5 from the 5' end in the seed region, and thus can achieve G:U and G:A wobble base pairing. Accordingly, as a result of analyzing the G:A and G:U wobble base pairing by the G bases at the specific sites, separately, as shown in FIG. 4B, the largest number (1,992) of canonical seed target sites (seed sites) of miR-124 were found. However, as compared with the above, 1800 sites with a G:U wobble pair at the base in position 4 (4G:A sites) were found, and then 1,542 sites with a G:A wobble pair were observed. In addition, 1,427 target sites with a G:U wobble pair and 1,196 target sites with a G:A wobble pair (5G:A sites) were observed. It was able to be confirmed that all of the investigated sites are present in a number greater than the number of the control (647 sites). The sequences and base pairs of the seed site, 4G:A sites and 5G:A sites for miR-124 are shown in FIG. 4C. Accordingly, when recognizing target mRNA with the base sequence of the seed region, miRNA allows canonical base pairing using the seed region as well as non-canonical G:U or G:A wobble base pairing, and it was able to be seen that through these types of pairing, miRNA can bind with target mRNA. Such miRNA target recognition through G:A wobble base pairing is a novel result that has not been reported yet, and as observed above, it was able to be confirmed that non-canonical targets are recognized in many miRNAs.

### 2-2. Discovery of non-canonical G:A wobble pair site of miR-1 by Ago HITS-CLIP assay on cardiac tissue of cardiomyopathy patient

In Example 2-1, G:A wobble base pairs between the miRNA seed region and target mRNA were analyzed from the Ago HITS-CLIP data obtained from human brain tissue, revealing that such pairs are present as non-canonical targets. Similarly, through analysis of Ago HITS-CLIP data obtained from cardiac tissues of six cardiomyopathy patients (FIG. 5A), whether G:A wobble base pairing is present in an miRNA target was examined. Here, the RNA sequencing result was bound with Argonaute-miRNA paired to a human miRNA sequence, thereby performing analysis by focusing on miR-1 specifically found in myocardial tissue among miRNAs frequently binding with the Argonaute protein in the corresponding cardiac tissue and focusing on G:A wobble base pairing of the corresponding seed region. Particularly, since the miR-1 seed region has three G bases in positions 2, 3 and 7 from the 5' end, these three G bases may pair with A, thereby binding to mRNA of a target gene, along with Argonaute.

Therefore, as a result of analyzing G:A wobble base pairs at the specific G sites, separately, as shown in FIG. 5B, it was confirmed that the number of G:A wobble pairs at the G in position 2 was 27, the number of G:A wobble pairs at the G in position 3 was 19, and the number of G:A wobble pairs at G in position 7 was 26, and all of the sites examined as above are present in a number greater than the number of the control (9 sites). In addition, like the previous report, the largest number (128) of canonical seed target sites of miR-1 were found. Accordingly, miR-1, which is most highly expressed in myocardial cells, allows not only canonical base pairing using the seed region, but also non-canonical G:A wobble base pairing in target mRNA recognition via the base sequence of the seed region, showing that, through such types of pairing, miRNA can bind with target mRNA.

According to the above-described example, it was confirmed that miRNA binds with G:A wobble pair sites non-canonically, thereby suppressing target gene expression. If conventional binding of target mRNA with A through wobble pairing by G in an miRNA seed sequence changes the miRNA function, it is expected that this mechanism will be shown through miRNA sequence variation in the case of a disease, and accordingly, sequencing of miRNA binding with Argonaute from the Ago HITS-CLIP result from cardiac tissue of cardiomyopathy patients was conducted to confirm whether G of miR-1 is changed to T. As a result of analyzing all variations occurring at 1^{st} to 20^{th} bases from the 5' end of miR-1 in the seed region of miR-1, as shown in FIG. 5C, it was observed that the G bases in positions 2, 3, 7, 12 and 15 from the 5' end of miR-1 are changed to T bases. Particularly, the fact that the G bases in positions 2, 3 and 7 in the seed region are changed to T nucleotides with higher frequency than other variations and sequenced indicates that, in the case of cardiomyopathy, G is changed to U, thereby changing an existing non-canonical G:A wobble pair into an existing canonical seed target site, rather than that existing miR-1 sequence pairs with target mRNA through non-canonical G:A wobble pairing when G at the corresponding site recognizes mRNA of a target gene.

### 2-3. Confirmation of inhibition of target gene expression through G:A wobble pairing in miR-1 seed sequence in cardiomyocytes

After, it was discovered that miRNA can bind to target mRNA by allowing non-canonical G:A wobble pairing in the seed region in Examples 2-1 and 2-2, a reporter experiment was conducted to confirm whether such binding can actually cause target gene suppression at a cellular level. Here, the experiment was conducted on miR-1 which is known to be highly expressed in muscle cells such as a cardiomyoblast cell line (H9c2) and was identified in Example 2-2, and particularly, conducted on G:A wobble pairs base pairing, focusing on G in position 7 from the 5' end of miR-1. To measure miRNA-mediated gene suppression more precisely at individual cell level, a fluorescent protein expression reporter system was constructed and used. The fluorescent protein expression reporter system was designed to express two colors of fluorescent proteins in one vector, and allowed a green fluorescent protein (GFP) to be expressed in an SV40 promoter, and several miRNA target sites were consecutively arranged in the 3' untranslated region (3' UTR) so as to measure a miRNA-mediated gene suppression effect by GFP intensity. At the same time, a red fluorescent protein (RFP) was allowed to be expressed by a Tk promoter, and the green fluorescent signal was normalized to the RF intensity (FIG. 6A).

A reporter (GFP-7G:A site) was formed by inserting a non-canonical target site (7G:A-site; 5'p-AAAUUCCA-3', SEQ ID NO: 2), which is complementary to bases in positions 2 to 8 from the 5' end of miR-1 and pairing with G in position 7 through G:A wobble pairing, into the fluorescent protein expression reporter vector formed as above, and then an experiment was conducted to confirm the reporter is inhibited by miR-1. Here, the experiment was performed by co-transfection of 500 ng of the GFP-7G:A site reporter vector and 25 nM miR-1 into a cardiomyoblast cell line (H9c2) using Lipofectamine 2000 (Invitrogen) according to the manufacturer's protocol, and 24 hours after transfection, each fluorescence signal was measured using a flow cytometer (Attune NxT, Life Technology).

As a result, as shown in FIG. 6A, the fact that the 7G:A site of miR-1 is very efficiently inhibited by miR-1 expression was confirmed compared with cells into which a control nucleotide (cont; NT) was introduced. That is, as a result of analyzing four parts (Q5-1, Q5-2, Q5-3 and Q5-4) according to the expression levels of two types of fluorescent proteins in the cardiomyoblast cell line (H9c2), it was observed that, in the miR-1-introduced cells, compared with the control nucleotide-introduced cells, the distribution of cells expressing a red fluorescent protein with an intensity of 10³ and a green fluorescent protein with an intensity of 10³ is reduced from 59.51% (control: Q5-3) to 41.80 % (miR-1: Q5-3).

From the above result, it was observed in the control that the GFP-7G:A site reporter vector is inhibited to a certain extent in H9c2 cells without miR-1 introduction. It can be estimated that the GFP-7G:A site reporter vector is inhibited by G:A wobble base pairing with the reporter target mRNA by miR-1 already expressed in H9c2 cells. To confirm the finding described above, a miRIDIAN microRNA Hairpin inhibitor (miR-1 inhibitor) capable of suppressing miR-1 expression in H9c2 cells was purchased from Dharmacon, and then used to transfect the cells at a concentration of 50 nM.

As a result, compared with a control, when the miR-1 inhibitor is introduced, the distribution of cells expressing a red fluorescent protein with an intensity of 10³ and a green fluorescent protein with an intensity of 10³ is increased to 81.56% (miR-1 inhibitor: Q5-3), confirming that miR-1 present in the cells can suppress gene expression due to the 7G:A site of miR-1.

In addition, a control (GFP-no site) in which an miRNA target site was not introduced into a fluorescent protein expression reporter or a GFP-7G:A site, which is a reporter in which a 7G:A site of miR-1 was introduced, was introduced into H9c2 cells, and their activities were compared. A positive control, in which miRNA target site was co-introduced with miR-1, was used for quantitative comparison. Here, the analysis was conducted by dividing RFP values measured by a flow cytometer by section and averaging the GFP values and then converting the average value to a log ratio, and the log ratio was obtained by setting the green fluorescent protein value of the GFP-no site (relative log GFP), which is the control, to 1.

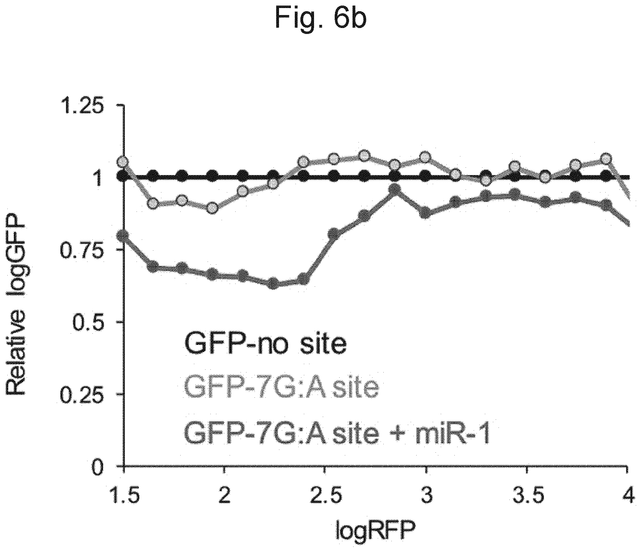

As a result, as shown in FIG. 6B, in the case of a site complementarily base pairing to G in position 7 of miR-1 through G:A wobble pairing (GFP-7G:A site), and particularly, in a section showing a red fluorescent protein expression (log RFP) of 1.5 to 2.5, it was confirmed that green fluorescent protein expression (relative log GFP) through G:A wobble pairing was approximately 12.5% lower than 1. In the positive control, it was observed that such inhibition is shown in the same pattern, in particular, it was observed that GFP expression (relative log GFP) was inhibited by approximately 40 to 25% in the section showing 1.5 to 2.5 of RFP expression (log RFP in the same pattern as) the inhibition pattern.

To confirm that the above-described results are not affected by differences in strengths of the different promoters and fluorescence activity of the two different fluorescent proteins, which were used in the corresponding fluorescent protein reporters, two fluorescent protein reporters, p.UTA.3.0, having a different fluorescent protein were purchased from Addgene (plasmid #82447). Here, the reporter experiment was conducted by constructing a reporter vector (RFP-7G:A site) by inserting a 7G:A sequence of miR-1 into the 3' UTR of RFP, regulated by an SV40 promoter, in the p.UTA.3.0 vector five times, and here, an RFP expression level was modified through normalization of a degree of a GFP value expressed by a Tk promoter, which reflects the degree of introduction into cells.

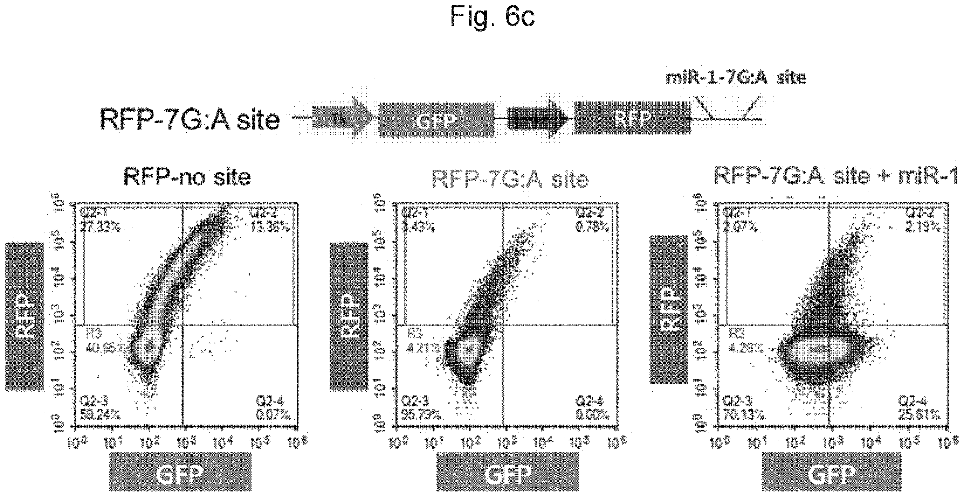

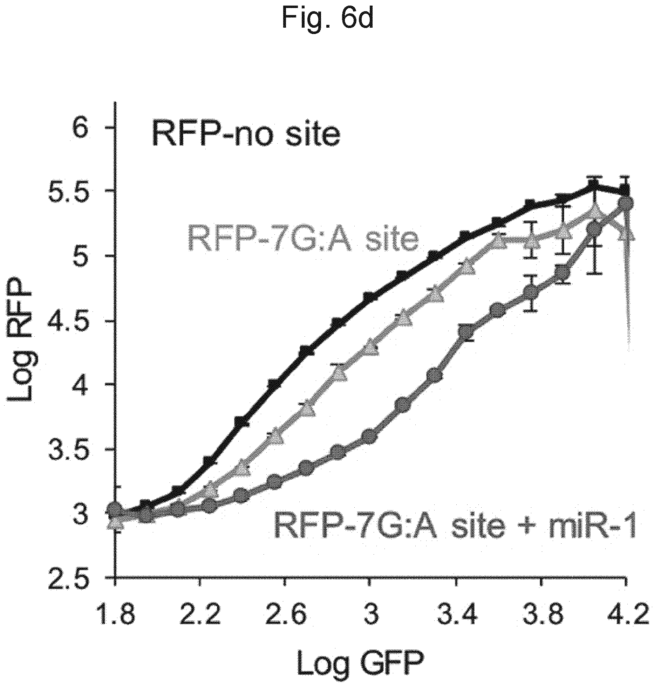

As a result, as shown in FIG. 6C, as described above, the suppression of the 7G:A site of miR-1 by miR-1 expressed in H9c2 cells was confirmed by comparing the results of a control, such as a reporter having no miRNA target site (RFP-no site), and a reporter having the 7G:A site of miR-1 (RFP-7G:A site), and at the same time, as shown in FIG. 6D, even by co-transfection of a positive control and miR-1, the suppression of the miR-1-7G:A site was also observed, as shown in FIG. 6B.

From above, it was confirmed that the non-canonical target of miRNA, which was found through Ago HITS-CLIP assay, may not only actually bind to the seed sequence of miRNA through G:A wobble pairing, but also suppress the expression of the corresponding target gene.

Therefore, considering this, sufficient binding with miRNA may be achieved by using the target sites through G:A wobble pairing, and it was able to be inferred that, if such binding is competitively made, and thus can suppress miRNA, only the biological function of miRNA can be suppressed by non-canonical target suppression through G:A wobble pairing.

### 2-4. Confirmation of suppression of expression of non-canonical nucleation bulge site of miRNA by luciferase reporter

miRNA is known to non-canonically recognize a target, other than canonical target recognition through consecutive base pairing of a seed region, and particularly, it was reported that, when the seed region of the miRNA is paired with target mRNA, a form of target mRNA where nucleotides in positions 5 and 6 from the 5' end of miRNA are bulged out is present. Particularly, in a nucleation bulge site found in miR-124, it was confirmed that a bulge was formed at G between positions 5 and 6, but it was not known how much the target inhibits the expression of the target gene, compared with a canonical target through the conventionally known seed pairing.

To confirm whether a base sequence for bulge formation may bind with miRNA to an extent to be functionally activated, an investigation was performed using a luciferase reporter. A canonical target seed site used herein is, as shown in the upper diagram of FIG. 7A, 5'-GUGCCUU-3' (miR-124-seed site, SEQ ID NO: 8) completely complementary to 5'-AAGGCAC-3', which is the sequence of bases in positions 2 to 7 from the 5' end of a seed sequence of miRNA-124 (5' UAAGGCACGCGGUGAAUGCCAA-3', SEQ ID NO: 7), and was inserted twice consecutively into 3' UTR of the *Renilla* luciferase gene in a luciferase reporter vector (psi-check2, Promega). In addition, as shown in the lower diagram of FIG. 7A, a bulge site where a base sequence that is able to complementarily pair with the 6^{th} base from the 5' end is formed in a bulge between positions 5 and 6 of a seed site target has the base sequence of 5'-GUGGCCUU-3' (miR-124-bulge site, SEQ ID NO: 9) in miR-124, and paired to 3'UTR of the *Renilla* luciferase gene consecutively twice in the same manner as described above, thereby constructing a reporter vector.

The luciferase reporter vector produced by the above-described method was co-transfected with various concentrations (0.02, 0.1, 0.5, 2.5 and 15 nM) of miR-124 into HeLa cells (ATCC CCL-2) using the Lipofectamine 2000 reagent (Invitrogen) according to the manufacturer's protocol to investigate its effect. The miR-124 used herein was chemically synthesized by Bioneer with two nucleic acid sequences including a guide strand and a passenger strand, separated by HPLC, and prepared in a duplex according to the method provided by the manufacturer. The HeLa cells were incubated in a Dulbecco's modified Eagle's medium (Invitrogen) supplemented with 10% FBS, 100 U/ml penicillin and 100 µg/ml streptomycin, and during transfection, the cells were incubated in an antibiotic-free complete medium. 24 hours after transfection, luciferase activity was measured using a dual-luciferase reporter assay system (Promega) according to the manufacturer's protocol to investigate the effect of miR-124, and the measurement of *Renilla* luciferase activity was repeatedly performed at least three times using a Glomax Luminometer (Promega), and calculated by normalization by the activity of firefly luciferase. The inhibitory effect of a nucleation bulge site was measured with the various concentrations of miR-124, and as a result of comparison with a canonical seed site of miR-124, as shown in FIG. 7B, the inhibitory concentration 50 (IC₅₀) of the nucleation bulge site and the seed target were 0.8 nM and 0.4 nM, respectively, and it was able to be observed that although the efficiency of inhibiting gene expression through the nucleation bulge site is 2-fold lower than the seed target, still, the miR-124 well inhibited a non-canonical target such as a nucleation bulge site.

From above, through the Ago HITS-CLIP assay, it was able to be seen that a nucleation bulge site previously reported as a non-canonical target does not only bind with miRNA, but also efficiently inhibits the expression of a gene having this site. In addition, considering this, it was able to be inferred that sufficient binding with miRNA may be made using the nucleation bulge site, and if such binding is competitively made and thus can inhibit miRNA, only the biological function of miRNA can be inhibited by the suppression of the nucleation bulge site.

### Examples 3. Confirmation of possibility of regulating another biological function through suppression of G:A wobble pair target in seed sequence of miRNA

### 3-1. Experimental materials and methods

In Example 2, Ago HITS-CLIP data obtained from human brain tissue and cardiac tissue of a cardiomyopathy patient revealed that G:A wobble base pairs between the seed region of miRNA and target mRNA are present as non-canonical targets, and according to an additional reporter experiment, it was confirmed that such a non-canonical target site can exhibit an miRNA-mediated gene suppression effect. The non-canonical gene suppression phenomenon suggested the possibility that G:A wobble base pairs act as non-canonical targets of miRNA to regulate the biological function of miRNA, and thus, to confirm this possibility, an experiment was conducted focusing on miR-1, which is miRNA of a cardiomyocyte, described in the above-described example. To investigate whether the non-canonical G:A wobble target has a specific biological function, since miR-1 has to recognize only a non-canonical G:A wobble pair target site and not recognize a canonical target, it is manufactured by substituting G with U, such that G in the seed region of miR-1 pairs with A, not C, to be used in the experiment. To perform this experiment under conditions physiologically similar to the heart, primary cardiomyocytes derived from cardiac tissue of a rat were cultured (primary rat neonatal cardiomyocyte culture). Here, the cardiomyocyte culture was performed by separating myocardial cells from rat neonatal cardiac tissue at one day after birth through an enzyme reaction (Neomyt kit (Cellutron)), and culturing the cardiomyocytes in a brdU-added medium prepared by mixing Dulbecco's modified Eagle's medium (Invitrogen) supplemented with 10% FBS, 5% horse serum (HS), 100 U/ml penicillin and 100 µg/ml streptomycin and M199 medium (WellGene) at 4:1, separately from other cells having a cell cycle, which are present in cardiac tissue.

### 3-2. Confirmation of size reduction in cardiomyocytes by miR-1

It was first confirmed whether cardiac hypertrophy is induced in the primary-cultured cardiomyocytes obtained as above. Cardiomyocytes have characteristics of an arrested cell cycle and an increased size, which is called cardiac hypertrophy. Cardiac hypertrophy plays a role in compensating for the decline of cardiomyocyte functions, is known as an intermediate stage of the pathology of heart disease, and is a heart disease model system with well-known cell morphology and changes in gene expression profile. In a cardiac hypertrophy cell model, cardiac hypertrophy may be induced by treating cardiomyocytes with an adrenaline receptor agonist such as phenylephrine (PE).

Accordingly, cardiac hypertrophy was induced by treating the cultured cardiomyocytes with 100 µM phenylephrine (+PE), and as shown in FIG. 8A, it was morphologically confirmed that the size of cardiomyocytes increased, compared with a control (rCMC) which was not treated. In addition, as shown in Example 1, when miR-1 was expressed in a cardiomyoblast cell line (H9c2 cells), cell size reduction occurs, and even when miR-1 was introduced into the culture primary cardiomyocytes, it was observed that the size of the cardiomyocytes was reduced due to the same phenomenon as above. This is consistent with the well-known cardiac hypertrophy phenomenon and the miR-1-induced myocardial atrophy in cardiomyocytes.

### 3-3. Confirmation of possibility of another biological function exhibited by G:A wobble pair target inhibition in seed sequence of miRNA using miR-1 substitute

Since miR-1 contains three G bases in a seed region, it does not recognize a canonical target, and to recognize only a non-canonical G:A wobble pair target site, miR-1 in which these G bases are substituted with U bases was designed, and miR-1 substitutes were synthesized by applying the miR-1 to position 2 (miR-1-G2U), position 3 (miR-1-G3U) and position 7 (miR-1-G7U) from the 5' end depending on a G site. The base sequences of the miR-1 substitutes used herein are as follows (miR-1-G2U: 5' p-UUGAAUGUAAAGAAGUAUGUAU-3'(SEQ ID NO: 10); miR-1-G3U: 5' p-UGUAAUGUAAAGAAGUAUGUAU-3'(SEQ ID NO: 11); and miR-1-G7U: 5' p-UGGAAUUUAAAGAAGUAUGUAU-3'(SEQ ID NO: 12)), which were synthesized with a guide strand, and a passenger strand (hsa-miR-1-5p; miRBase Accession No: MIMAT0031892, 5'-ACAUACUUCUUUAUAUGCCCAU-3', SEQ ID NO: 13) which is not completely complementary to the 2^{nd}, 3^{rd}, and 7^{th} bases from the 5' end of the substituted miR-1 and forms a duplex with a guide strand, was chemically synthesized by Bioneer after a human miR-1 base sequence recorded in miRBase was designed, separated by HPLC, and then prepared in a duplex of a guide strand and a passenger strand according to the method provided by the manufacturer. The transfection of 50 mM of the corresponding miRNA into the primary myocardial cell line was performed using a RNAimax reagent (Invitrogen).

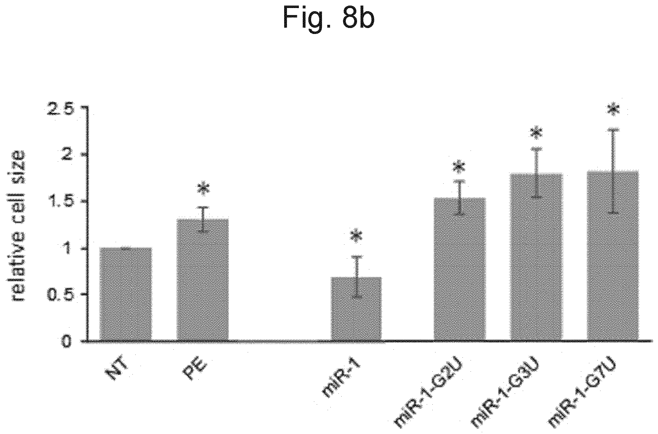

As a result, as shown in FIG. 8A, when miR-1-G2U, miR-1-G3U or miR-1-G7U was introduced in the primary cardiomyocyte culture, it was able to be observed that the morphology of a cardiomyocyte is increased to a similar level of cardiac hypertrophy-induced cells (+PE). To quantitatively analyze each cell size, 100 or more cells were quantified using the ImageJ program (NIH), and as shown in FIG. 8B, it was analyzed that all of the miR-1 substitutes (miR-1-G2U, miR-1-G3U and miR-1-G7U) synthesized to recognize only a G:A wobble pair site of miR-1 are approximately 1.5 to 1.8-fold increased in size, compared with the case of introduction of a control nucleotide (NT). This is similar to the cardiomyocyte size of a phenylephrine (PE)-induced cardiomyocytes hypertrophy model, and miR-1 itself shows an effect of reducing a cell size in terms of the morphology of cardiomyocytes, and thus it was observed that the canonical target inhibitory effect leads to a different cell morphological function from target inhibition through G:A wobble pairing. In addition, to additionally confirm the cardiomyocyte hypertrophy observed in this example at a molecular level, the expression of an atrial natriuretic peptide (ANP) gene known to increase its expression as a marker was compared with GAPDH through quantitative polymerase chain reaction (qPCR) and measured as a relative value. The experiment was performed by extracting total RNA with a RNeasy kit (Qiagen), reverse-transcribing the total RNA with Superscript III RT (Invitrogen) according to the corresponding protocol, amplifying mRNA of ANP with corresponding primers through qPCR using SYBR™ Green PCR Master Mix (Applied Biosystems), and measuring the amplified amount.

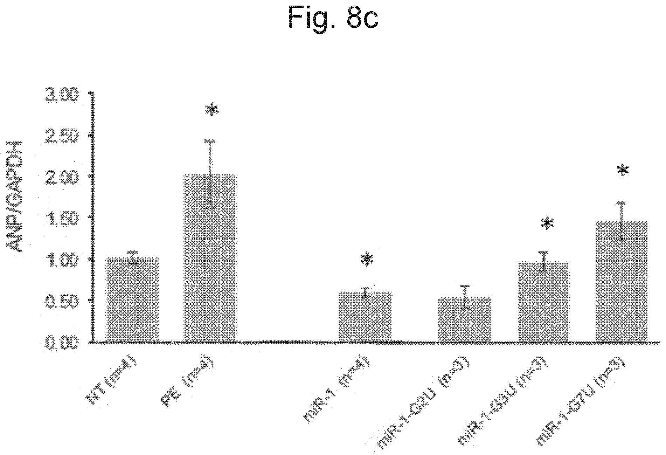

As a result, as shown in FIG. 8C, when the miR-1 substitutes (miR-1-G2U, miR-1-G3U and miR-1-G7U) synthesized to recognize only the G:A wobble pair site of miR-1 were introduced into cardiomyocytes, all of the substitutes had a significantly increased ANP expression level which was approximately 1.2 to 1.5-fold higher than that of the control (NT), which was confirmed by four-repeated experiments. Although such increases in ANP expression are less than that in the phenylephrine (PE)-treated cardiomyocyte experimental group, which is approximately 2 fold higher than that of the control, it is opposite of the phenomenon of significantly reducing ANP expression even when the original miR-1 was introduced, indicating that miR-1 has a completely different biological function through a G:A wobble pair site.

From the above, it was able to be finally known that the non-canonical target suppressed by G:A wobble pairing in the miRNA seed region has a biologically different function from the conventional canonical target recognition. Such finding shows that the function through the canonical target of miRNA is different from the function of the non-canonical target, and considering this, it was expected that, if only the G:A wobble target of miRNA is able to be suppressed, only the biological function occurring by a G:A wobble target will be selectively controlled.

### Example 4. Confirmation of effect of inhibiting non-canonical G:A wobble pairing function of miRNA by tandem-target miRNA inhibitor

### 4-1. Confirmation of effect of tandem-target miRNA inhibitor (2x) effect on non-canonical G:A seed-binding site of miR-1

The anti-miR-1-seed(2x) (5'p-dTdT (rSP) ACAUUCCA (rSP) ACAUUCCA (rSP)dTdT-3'), which is the tandem-target miRNA inhibitor formed to inhibit the seed target site, which is the canonical target of miR-1, in Example 1, showed a phenomenon of inhibiting the miR-1 function of reducing the size of cardiomyocytes. Therefore, the tandem-target miRNA inhibitor can be applied to any sequence that is able to bind with miRNA, and thus also applied to the non-canonical target sequences of miRNA identified in Examples 2 and 3. Accordingly, to suppress only the biological functions of the G:A wobble site confirmed in Examples 2 and 3, the tandem-target miRNA inhibitor was applied to miR-1.

First, like the sequence shown in FIG. 9A, two sequences complementary to the sequence in which G in position 7 from the 5' end is substituted with U in the miR-1 seed sequence were introduced into the tandem-target miRNA inhibitor (anti-miR-1-7G:A(2x)). That is, the tandem-target miRNA inhibitor (anti-miR-1-7G:A(2x)) was designed by consecutively arranging making complementary base pairing of 5' -AAAUUCCA-3' capable of complementarily base pairing with the seed sequence for the miR-1-G7U of Example 3, and introducing rSpacer which is an abasic nucleotide, as shown in Example 1, between the arranged sequences to prevent an off-target effect caused by the creation of a new site on the boundary between the consecutively arranged sequences.

To experimentally confirm the efficiency of suppressing the non-canonical G:A wobble seed target gene of miR-1, as described in Example 1-2, a luciferase reporter assay was performed. To this end, as shown in FIG. 9B, 5 non-canonical G:A wobble seed target sites (miR-1-7 G:A site) of miR-1 were consecutively arranged in the 3'UTR of the luciferase reporter gene (Luc-miR-1-7G:A site).

To confirm whether a gene is reliably suppressed by the non-canonical G:A wobble seed target site of miR-1, and then such suppression is inhibited by a corresponding chain target miR-1 G:A wobble site inhibitor (anti-miR-1-7G:A(2x)), miR-1:G7U in which the G in position 7 from the 5' end of miR-1 is substituted with U was synthesized by Bioneer according to the same method as described in the above Example and used at 5 nM, and the miR-1:G7U was co-transfected with 50 nM of the inhibitor (anti-miR-1-7G:A (2x), SEQ ID NO: 14) into HeLa cells (ATCC CCL-2) using the Lipofectamine 3000 reagent (Invitrogen) according to the manufacturer's protocol to investigate whether the anti-miR-1-7G:A(2x) exhibits an effect of inhibiting the suppression of the non-canonical G:A wobble seed target.

As a result, as shown in FIG. 9C, it was confirmed that the luciferase activity observed after miR-1:G7U transfection is inhibited to be 88% of relative luciferase activity (%) calculated based on the value when the miR-1:G7U is not transfected, and relative luciferase activity (%) of an experimental group into which miR-1 and anti-miR-1-7G:A (2x) are introduced is 95%, confirming that the activity of luciferase whose expression is inhibited by miR-1:G7U is almost restored to the original value which is not inhibited, and is statistically significant (p=0.027).

### 4-2. Confirmation that tandem-target miRNA inhibitor (2x) can inhibit biological function caused by non-canonical G:A seed binding site of miR-1

The function of the anti-miR-1-7G:A(2x) specifically inhibiting the miR-1 function of regulating a 7G:A wobble target in the above example has been confirmed in HeLa cells (ATCC CCL-2) into which a miR-1-G7U-substitution base is transfected from the outside through the investigation of luciferase reporter activity. Accordingly, to confirm the 7G:A wobble target regulating function of miR-1 naturally present in cardiomyoblast cells, the Luc-miR-1-7G:A site, which the miR-1-7G:A site-contained luciferase reporter vector and 50 nM anti-miR-1-7G:A (2x) or control anti-NT (2x), which are used in the experiment in Example 4-1, were transfected, and then the luciferase activity was investigated as described in Example 4-1.

As a result, as shown in FIG. 10A, a relative activity (%) value calculated by normalizing the activity value of the miR-1-7G:A site-containing luciferase to an activity value of a luciferase reporter without a miR-1-7G:A site (Luc-no site) is 86.28+/-2.59, corresponding to a value measuring the 7G:A wobble target regulating function of miR-1 naturally present in cardiomyoblast cells. Here, the relative luciferase activity (%) of the anti-miR-1-7G-transfected experimental group is 89.96+/-2.13, showing that the activity of miR-1-G7U is statistically significantly inhibited.

It was shown that H9c2 cells, which is a cardiomyoblast cell line, naturally expresses miR-1 therein, can suppress a target gene through 7G:A wobble pairing of miR-1. In addition, in Example 3-3, it was observed that, in order to pair between the 7^{th} base of miR-1 with A, mi-1-G7U in which G in position 7 is substituted with U makes the size of cardiomyocytes larger because of its expression. Therefore, it was expected that, if a G:A wobble pair target through the G in position 7 of miR-1 in the H9c2 cells can be inhibited by a miRNA-inhibiting modified nucleic acid according to the present invention, the cardiomyocytes will be smaller, and to confirm this, as a result of transfecting a control (anti-NT(2x)) and anti-miR-1-7G:A(2x) into the H9c2 cells in the same manner as described in the above example and confirming the transfection effect, as shown in FIG. 10B, it was observed that the size of the H9c2 cells is reduced.

In addition, the sizes of 100 or more H9c2 cells were quantitatively analyzed using the ImageJ program (NIH), as shown in FIG. 10C, confirming that the size of anti-miR-1-7G:A(2x)-introduced cells is reduced by approximately 40% compared with a control (anti-NT(2x)). As seen from this, the anti-miR-1-7G:A(2x) was able to competitively inhibit a G:A wobble target recognition mechanism due to the G base in position 7 of miR-1, thereby inhibiting the corresponding function.

As described above, it can be seen that the tandem-target miRNA inhibitor may not only prevent the conventionally known canonical seed target recognition of miRNA, but also effectively inhibit the specific function of miRNA even when it is applied to a non-canonical target such as G:A wobble pair.

### 4-3. Confirmation that tandem-target microRNA inhibitor (9x) can inhibit biological function of miR-1 by non-canonical G:A seed binding site

The tandem-target miRNA inhibitor (anti-miR-1-7G:A(2x)) for the non-canonical G:A seed-binding site of miR-1 used in this example has two consecutive non-canonical G:A seed-binding sites of miR-1, which are to be specifically suppressed. Moreover, as shown in FIG. 11A, a tandem-target miRNA inhibitor (anti-miR-1-7G:A(9x)) in which the corresponding sites are repeated 9 times, was designed, and a luciferase reporter experiment for confirming the inhibitory effect thereof was conducted (FIG. 11B).

The anti-miR-1-7G:A(9x) was chemically synthesized with the following sequence by Trilink, and separated by HPLC (anti-miR-1-7G:A (9x): 5' -p-NN(rSP)AAAUUCCA(rSP)AAAUUCCA(rSP)AAAUUCCA(rSP)AAA UUCCA(rSP)AAA UUCCA(rSP)AAAUUCCA(rSP)AAAUUCCA(rSP)AAAUUCCA(rSP)AAAUUCCA(rSP) NN-3', SEQ ID NO: 15). The control inhibitor used in the experiment as the control was also chemically synthesized with the following base sequence by Trilink to have 9-repeated target sites of NT, and separated by HPLC (anti-NT (9x): 5' -p-NN(rSP)GGUUGUGA(rSP)GGUUGUGA(rSP)GGUUGUGA(rSP)GGUUGUGA(rSP)GGU UGUGA(rSP)GGUUGUGA(rSP)GGUUGUGA(rSP)GGUUGUGA(rSP)GGUUGUGA(rSP) NN-3', SEQ ID NO: 16). Here, miR-1-7G:A site-containing luciferase reporter vector Luc-miR-1-7G:A site and 25 or 50 nM anti-miR-1-7G:A (9x) or anti-NT (9x), which is a control, were transfected with 5 nM miR-1:G7U into HeLa cells (ATCC CCL-2) using the Lipofectamine 3000 reagent (Invitrogen) according to the manufacturer's protocol as used in the experiment of FIG. 9B, and luciferase activity was investigated.

As a result, as shown in FIG. 11B, it was observed that relative activity (%) calculated by normalizing a luciferase activity value of an experiment into which both of miR-1-G7U and anti-NT(9x) are transfected to the activity value of a luciferase reporter into which miR-1-G7U was not transfected is 74.57+/-6.36, thereby confirming the effect of the corresponding miRNA-mediated gene suppression. Here, it was observed that, in the experimental group into which the anti-miR-1-7G:A (9x) was also introduced, the relative activity (%) is 87.23+/- 5.68, indicating that 25 nM anti-miR-1-7G:A (9x) statistically significantly inhibits the activity of miR-1-G7U (P=0.05). In addition, when the amount of the anti-miR-1-7G:A (9x) was increased to 50 nM, the relative activity (%) of the luciferase reporter is 107.15 +/- 9.58, which statistically significantly shows that the target gene suppression is completely inhibited by miR-1-G7U (P=0.0008).

Therefore, it was able to be seen that the inhibitory effect of the tandem-target miRNA inhibitor can be detected when the target base sequence was repeated twice or more, but when repeated 9 times, a further increased inhibitory effect was exhibited.

The effective inhibition of anti-miR-1-7G:A (9x) to suppress a 7G:A wobble pairing target of miR-1 was confirmed using a luciferase reporter, and then it was confirmed that the anti-miR-1-7G:A (9x) can also effectively inhibit the cardiomyocyte atrophy phenomenon shown by suppressing the 7G:A wobble seed target of miR-1. Here, for the experiment, H9c2 cells, which is a cardiomyoblast cell line, were used, the change in cell size due to the anti-miR-1-7G:A (9x) was investigated over time (0, 24, 48 and 60 hours) after transfection using Lumascope 400 (Etaluma). In the corresponding experiment, the introduction of the anti-miR-1-7G:A was observed by removing FBS during transfection and treating the cells with phenylephine (PE; Sigma), which is a 100 uM alpha-adrenergic agonist, and culturing the cells for up to 60 hours.

As a result, as shown in FIG. 11C, the increase in cell size of the cardiomyoblast cell line induced by PE treatment was observed in the control (anti-NT(9x)), whereas in the experimental group into which the anti-miR-1-7G:A (9x) was transfected, the atrophy of the cardiomyoblast cell line was not observed at all.

As described above, as the number of miRNA targets to be suppressed increases, the inhibitory effect of the tandem-target miRNA inhibitor is increased, indicating that such inhibitory ability is strong enough to inhibit the biological function of the corresponding miRNA.

### Example 5. Confirmation that adverse effect caused by linker sequence between target sites is eliminated through introduction of spacer modifier into tandem-target miRNA inhibitor

The tandem-target miRNA inhibitor prepared in the above example was designed to consecutively align two or more miRNA target sites to be suppressed. Accordingly, the tandem-target miRNA inhibitor may inadvertently bind with different miRNAs or RNA-binding proteins due to a linker sequence between the target sites, and thus may have an adverse effect of inhibiting the inhibitor. In this case, in the sequence of an inhibitor (anti-miR-1-7G:A(9X)) suppressing a non-canonical G:A seed target by the G in position 7 of miR-1, for example, the base sequence of the anti-miR-1-7G:A (9x)) has an abasic modifier, rSpacer, between the repeated target sites as shown in FIG. 12A, and the corresponding sequence is as follows (5'-p-NN(rSP)AAAUUCCA(rSP)AAAUUCCA(rSP)AAAUUCCA(rSP)AAAUUCCA(rSP)AAA UUCCA(rSP)AAAUUCCA(rSP)AAAUUCCA(rSP)AAAUUCCA(rSP)AAAUUCCA(rSP) NN-3'). In contrast, the anti-miR-1-7G:A(9x)-NS has the same base sequence, but not containing an abasic modifier between the repeated target site sequences, and the base sequence thereof is as follows (5'-NNAAAUUCCAAAAUUCCAAAAUUCCAAAAUUCCAAAAUUCCAAAAUUCCAAA AUUCCAAAAUUCCAAAAUUCCANN-3', SEQ ID NO: 17).

Here, as shown in FIG 12A, the only base sequence capable of complementarily binding in consecutive pairing is 5'-AAAUUCCA-3', but in the case of anti-miR-1-7G:A(9x)-NS having no abasic modifier, sequences capable of binding with miRNA are inadvertently produced by 7 sites which are linked with each other, in addition to the original target site, that is, 5'-AAAUUCCA-3', and these base sequences are as follows (5'-AAUUCCAA-3', 5'-AUUCCAAA-3', 5'-UUCCAAAA-3', 5'-UCCAAAAU-3', 5'-CCAAAAUU-3', 5'-CAAAAUUC-3', and 5'-AAAAUUCC-3'). Accordingly, all tandem-target miRNA inhibitors used in the example of the present invention are modified with the abasic modifier, rSpacer, between target sites, which is for preventing a new sequence itself generated by a linker sequence between the target sites, from accidentally being a target site of different miRNAs (miR-junction), or from being a binding site of a different RNA-binding protein, as shown in FIG. 12B.

Therefore, to confirm whether the tandem-target miRNA inhibitor of the present invention prevents the above-described adverse effects by an abasic modifier, a luciferase reporter experiment was performed in the same manner as described in the above example. To confirm this, miR-junction, which is miRNA recognizing a linker sequence between target sites, was synthesized with the sequence shown in FIG.12B. This includes bases, which are completely complementary to a linker sequence between the target sites, present in positions 1 to 8 from the 5' end, and a sequence of Cel-miR-67-3p in positions 9 to 21 (miR-junction: 5'-AAAUUUUG CCUAGAAAGAGUA -3', SEQ ID NO: 18). To perform the experiment with a double polymer, miR-junction was chemically synthesized by Bioneer with a passenger strand (miR-junction-passenger: 5'-TACTCTTTCTAGGCAAAATTT-3'), and separated by HPLC. In addition, to measure the inhibitory effect of the corresponding miR-junction, the target site (5'-CAAAATT-3'), which is a luciferase reporter vector was constructed to have five repeated base sequence complementary to the bases in positions 1 to 8 of the miR-junction, and used by the same method as described in the above example.

As a result of the luciferase reporter experiment to examine the inhibitory effect by the miR-1 junction, as shown in FIG. 12C, in the case of anti-miR-1-7G:A (9X) having a spacer modifier between target sites, since the corresponding target site is divided by rSpacer, the inhibition of the gene suppression effect by up to 12% due to the expression of 5 nM of the MiR-1 junction was not statistically significant (P=0.23), but in the case of anti-miR-1-7G:A(9X)-NS not having a spacer modifier, the inhibition of the gene suppression by the miR-1 junction is statistically significant (P=0.01).

As described above, it was able to be confirmed that, since the tandem-target miRNA inhibitor has an abasic modifier between target sites of miRNA to be suppressed, the adverse effects caused by the linker sequence between sites can be eliminated.

As above, as specific parts of the specification have been described in detail, although it is clear to those skilled in the art that this specific technique is merely a preferred embodiment, the scope of the specification is not limited thereto. Thus, the substantial scope of the specification will be defined by the accompanying claims and their equivalents.

### [Industrial Applicability]

A modified nucleic acid inhibiting miRNA according to the present invention contains an abasic spacer introduced between miRNA target sites, thereby not only efficiently suppressing miRNA, but also preventing inadvertent binding of the sequence introduced into the modified nucleic acid with different miRNAs. Therefore, the modified nucleic acid is expected to be effectively used to selectively suppress only a biological function caused by the non-canonical target recognition of miRNA.

## Claims

1. A modified nucleic acid inhibiting miRNA, comprising:
at least two consecutive miRNA target site sequences, each consisting of at least 6 nucleotides,
wherein the miRNA target site sequence is one selected from the group consisting of
1) a sequence complementary to bases in positions 2 to 7 or 3 to 8 from the 5' end of miRNA,
2) a sequence complementary to bases in positions 2 to 7 from the 5' end of miRNA and having a bulge between the bases in positions 5 and 6, and
3) a sequence complementary to bases in positions 2 to 7 or 3 to 8 from the 5' end of miRNA, and having complementary G:A or G:U wobble base pairs, and
at least one base is substituted with an abasic spacer between the miRNA target site sequences.

2. The modified nucleic acid of claim 1, wherein the miRNA is miR-1.

3. The modified nucleic acid of claim 1, wherein the modified nucleic acid inhibiting miRNA contains a 5'-ACAUUCCA-3' or 5'-AAAUUCCA-3' sequence.

4. The modified nucleic acid of claim 3, wherein the modified nucleic acid inhibiting miRNA consecutively comprises at least two 5'-ACAUUCCA-3' sequences, and at least one abasic spacer before and after the sequence.

5. The modified nucleic acid of claim 3, wherein the modified nucleic acid inhibiting miRNA consecutively comprises at least two 5'-AAAUUCCA-3' sequences, and at least one abasic spacer before and after the sequence.

6. The modified nucleic acid of claim 1, wherein the miRNA target site sequence comprises 6 to 8 nucleotides complementary to a seed region of miRNA.

7. The modified nucleic acid of claim 6, wherein the seed region is a sequence between positions 1 to 8 from the 5' end of miRNA.

8. The modified nucleic acid of claim 1, wherein the abasic spacer is one or more selected from the group consisting of an abasic ribonucleotide spacer with no base (rSpacer, rSp), an abasic deoxyribonucleotide spacer (dSpacer), a C3 spacer and a C6 spacer.

9. A method of preparing a modified nucleic acid inhibiting miRNA, comprising:
consecutively arranging one selected from the group consisting of:
1) a sequence complementary to bases in positions 2 to 7 or 3 to 8 from the 5' end of miRNA,
2) a sequence complementary to bases in positions 2 to 7 from the 5' end of miRNA and having a bulge between the bases in positions 5 and 6, and
3) a sequence complementary to bases in positions 2 to 7 or 3 to 8 from the 5' end of miRNA, and having complementary G:A or G:U wobble base pairs; and
substituting at least one base with an abasic spacer in-between the consecutively arranged base sequence.

10. A method of inhibiting miRNA, which comprising:
introducing the modified nucleic acid inhibiting miRNA of any one of claims 1 to 8 into cells *in vitro.*

11. A gene transfer system comprising the modified nucleic acid inhibiting miRNA of any one of claims 1 to 8.


<110>     Korea University Industry and Academy Cooperation Foundation

<120>     Modified nucleic acids suppressing microRNA and use thereof

<130>     MPCT19-046

<150>     KR 10-2018-0063047
<151>     2018-05-31

<150>     KR 10-2019-0064767
<151>     2019-05-31

<160>     18

<170>     KoPatentIn 3.0

<210>     1
<211>     8
<212>     RNA
<213>     Artificial Sequence

<220>
<223>     anti-miR-1-seed site


<400>     1
acauucca                                                                 8


<210>     2
<211>     8
<212>     RNA
<213>     Artificial Sequence

<220>
<223>     anti-miR-1-7G:A seed site


<400>     2
aaauucca                                                                 8


<210>     3
<211>     23
<212>     RNA
<213>     Artificial Sequence

<220>
<223>     anti-miR-1-seed(2x): n is abasic rSpacer


<400>     3
ttnacauucc anacauucca ntt                                               23


<210>     4
<211>     22
<212>     RNA
<213>     Homo sapiens

<220>
<221>     gene
<222>     (1)..(22)

<223>    miR-1

<400>    4
uggaauguaa agaaguaugu au                                                22

<210>    5
<211>    24
<212>    RNA
<213>    Artificial Sequence

<220>
<223>    cel-miR-67

<400>    5
ucacaaccuc cuagaaagag uaga                                              24

<210>    6
<211>    23
<212>    RNA
<213>    Artificial Sequence

<220>
<223>    anti-NT (2x): n is abasic rSpacer

<400>    6
ttngguugug angguuguga ntt                                               23

<210>    7
<211>    22
<212>    RNA
<213>    Homo sapiens

<220>
<221>    gene
<222>    (1)..(22)
<223>    miRNA-124

<400>    7
uaaggcacgc ggugaaugcc aa                                                22

<210>    8
<211>    7
<212>    RNA
<213>    Artificial Sequence

<220>
<223>    miR-124-seed site

<400>    8
gugccuu                                                                  7

<210>    9
<211>    8

<212>    RNA
<213>    Artificial Sequence

<220>
<223>    miR-124-bulge site


<400>    9
guggccuu                                                                8


<210>    10
<211>    22
<212>    RNA
<213>    Artificial Sequence

<220>
<223>    miR-1-G2U


<400>    10
uugaauguaa agaaguaugu au                                                22


<210>    11
<211>    22
<212>    RNA
<213>    Artificial Sequence

<220>
<223>    miR-1-G3U


<400>    11
uguaauguaa agaaguaugu au                                                22


<210>    12
<211>    22
<212>    RNA
<213>    Artificial Sequence

<220>
<223>    miR-1-G7U


<400>    12
uggaauuuaa agaaguaugu au                                                22


<210>    13
<211>    22
<212>    RNA
<213>    Artificial Sequence

<220>
<223>    hsa-miR-1-5p


<400>    13
acauacuucu uuauaugccc au                                                22

<210>    14
<211>    23
<212>    RNA
<213>    Artificial Sequence

<220>
<223>    anti-miR-1-7G:A(2x): n is abasic rSpacer


<400>    14
ttnaaauucc anaaauucca ntt                                              23


<210>    15
<211>    82
<212>    RNA
<213>    Artificial Sequence

<220>
<223>    anti-miR-1-7G:A(9x): n is abasic rSpacer


<400>    15
naaauuccan aaauuccana aauuccanaa auuccanaaa uuccanaaau uccanaaauu      60

ccanaaauuc canaaauucc an                                               82


<210>    16
<211>    82
<212>    RNA
<213>    Artificial Sequence

<220>
<223>    anti-NT(9x): n is abasic rSpacer


<400>    16
ngguugugan gguugugang guugugangg uuguganggu ugugangguu gugangguug      60

ugangguugu gangguugug an                                               82


<210>    17
<211>    72
<212>    RNA
<213>    Artificial Sequence

<220>
<223>    anti-miR-1-7G:A(9x)-NS


<400>    17
aaauuccaaa auuccaaaau uccaaaauuc caaaauucca aaauuccaaa auuccaaaau      60

uccaaaauuc ca                                                          72


<210>    18
<211>    21
<212>    RNA
<213>    Artificial Sequence

<220>
<223>   miR-junction

<400>   18
aaauuuugcc uagaaagagu a                                                21